# EUROPEAN PATENT SPECIFICATION

(11) **EP 4 439 493 B1**
(45) Date of publication and mention of the grant of the patent: **09.09.2026**
(21) Application number: 23165026.8
(22) Date of filing: 29.03.2023
(51) Int. Cl.: G06V 20/69, G06V 10/82, G06N 3/045, G16C 20/70, G06N 3/08, G16B 15/30, G16B 40/20, G16B 40/30, G16C 20/30, G16C 20/50

(54) **METHODS, SYSTEMS AND COMPUTER PROGRAMS FOR PROCESSING IMAGES OF AN OPTICAL IMAGING DEVICE AND FOR TRAINING ONE OR MORE MACHINE-LEARNING MODELS**
VERFAHREN, SYSTEME UND COMPUTERPROGRAMME ZUR VERARBEITUNG VON BILDERN EINER OPTISCHEN BILDGEBUNGSVORRICHTUNG UND ZUM TRAINIEREN EINES ODER MEHRERER MASCHINENLERNMODELLE
PROCÉDÉS, SYSTÈMES ET PROGRAMMES INFORMATIQUES POUR TRAITER DES IMAGES D'UN DISPOSITIF D'IMAGERIE OPTIQUE ET POUR ENTRAÎNER UN OU PLUSIEURS MODÈLES D'APPRENTISSAGE AUTOMATIQUE

(43) Date of publication of application: 02.10.2024
(73) Proprietor: Leica Microsystems CMS GmbH, 35578 Wetzlar (DE)
(72) Inventor: KAPPEL, Constantin, 35578 Wetzlar (DE)
(74) Representative: 2SPL Patentanwälte PartG mbB

(56) References cited:
- US-A1- 2022 246 244
- SANCHEZ-FERNANDEZ ANA ET AL: "Contrastive Learning of Image and Structure-Based Representations in Drug Discovery", ICLR 2022 MACHINE LEARNING FOR DRUG DISCOVERY, 6 April 2022 (2022-04-06), pages 1 - 13, XP093064259, Retrieved from the Internet <URL:https://openreview.net/pdf?id=OdXKRtg1OG> [retrieved on 20230714]

## Description

### Technical field

Examples relate to a method, a system, and a computer program for processing images of an optical imaging device.

### Background

In the field of life-science microscopy, it is of interest to have highly specific labels or tags, which are molecules specifically attaching to particular structures in a specimen to create contrast.

For example, such a label or tag may be a low molecular weight chemical, which bears a fluorophore, which is a residue or part of the molecule that has electronic energy transitions of its molecular orbitals in the visible light spectrum and that readily undergoes reversible transitions upon excitation with light. Usually, these fluorophores (for simplification, the signifying part is being referred to in a *pars pro toto* fashion in lieu of the whole molecule) are covalently bound to an immunoglobulin (i.e., an antibody) or they selectively bind to e.g., cellular structures. A common trade name of such a label/tag is DAPI (4',6-diamidino-2-phe-nylindole), which is used to label DNA (Deoxyribonucleic Acid). Fluorophores can also be bound to oligonucleotides which then specifically hybridize to particular stretches of DNA to label, e.g., gene loci, a technique known as fluorescence in situ hybridization (FISH).

Alternatively, such a label or tag may be a polypeptide (i.e., protein) which spontaneously folds and undergoes chemical reaction on amino acid side-chains effectively creating a fluorophore inside the folded protein. A whole class of such fluorescent proteins exist, which are usually introduced into transgenic organisms by means of molecular biology (e.g., DNA fusing techniques) and then get expressed under an endogenous promotor or hyperexpressed under an exogenous promotor. Thus, an artificial fusion protein is created which carries the protein of interest and a fluorescent protein.

Alternatively, such a label or tag may be a low to mid molecular weight compound, which binds to a short peptide that is engineered as a fusion between protein of interest and "tagging" peptide. The peptide itself is not itself fluorescent, but a particular fluorophore can bind specifically to it to visualize the structure inside the specimen it attaches or localizes to.

All of the above are generally identified either by forward engineering, which is intrinsically difficult, or, more commonly, by generating a large number of candidate molecules that are then screened to select for appropriate properties. In the context of (biological) microscopy, this may result in the production of a large number of chemicals, polypeptides or polynucleotides (a "library"), which is then screened by staining or expressing each one in cell cultures, tissues or similar. In an emerging area, which is of clinical relevance, fluorescent markers are introduced into a patient undergoing surgery. In this case, such screenings are often not feasible, thus limiting the potential chemical space which can be covered to find new non-toxic tags with high quantum yield to generate contrast.

Sanchez-Fernandez et al: "Contrastive learning of image- and structure-based representations in drug discovery" relates to various techniques for machine learning in drug discovery. A first goal of the scientific paper is to train a machine-learning model, using unsupervised learning, to be able to generate a representation of a molecule regardless of whether a structure representation of the molecule is used as input or whether an image of the effect the molecule has on cells is used as an input. A second goal of the scientific paper is to make sure that the resulting representation is transferable, i.e., that the resulting representation can be used for subsequent activity prediction tasks.

There may be a desire for providing an improved concept for identifying molecules that can be used as labels or tags.

### Summary

The present invention is defined as set out in the appended claims.

Various examples of the present disclosure are based on the finding, that chemical properties of candidate molecules can be mapped into a semantic space, by computing an embedding of the molecule, which indicates a position of the candidate molecule in the semantic space. For example, a machine-learning model can be used to compute the embeddings based on tokenized representations of the molecule, and thus map the molecules into the semantic space. At the same time, knowledge, and in particular images, about existing molecules, such as molecules that are already used as tags/labels, may be used to train a machine-learning model to predict an embedding of a molecule based on the effect the molecule has on a biological sample. This enables the creation of a molecule evaluation pipeline, where a molecule with a known embedding is applied onto a biological sample, one or more images are taken of the biological sample (with the image(s) showing a target property exhibited by the molecule), and where the machine-learning model is used to predict the embedding of the molecule from the one or more images. If the known embedding and the predicted embedding are sufficiently similar, it is shown that the molecule behaves as expected with respect to the target property. This molecule evaluation pipeline can now be applied on candidate molecules that are expected to behave similar to known tags/labels. These candidate molecules can, for example, be determined by selecting molecules with an embedding that is similar to the embedding of a molecule that is a known label or tag. Thus, not only the validation of the target property of the candidate molecules can be performed in a largely automated fashion, but also a de novo selection of candidate molecules can be performed, in order to identify candidates of interest.

Some aspects of the present disclosure relate to a method for processing images of an optical imaging device. The method comprises obtaining embeddings of a plurality of candidate molecules. The method comprises obtaining, for each candidate molecule, one or more images of the optical imaging device. The one or more images show a visual representation of a target property exhibited by the candidate molecule in a biological sample. The method comprises processing, using a machine-learning model, for each candidate molecule, the one or more images and/or information derived from the one or more images to generate a predicted embedding of the candidate molecule. The machine-learning model is trained to output the predicted embedding for an input comprising the one or more images and/or the information derived from the one or more images. The method comprises comparing the embeddings of the candidate molecules with the predicted embeddings of the candidate molecules. The method comprises selecting one or more candidate molecules based on the comparison. A candidate molecule is selected if the embedding of the candidate molecule and the predicted embedding of the molecule are sufficiently similar, showing that the candidate molecule behaves as expected with respect to the target property. Using these techniques, candidate modules can be selected that behave as expected.

**In** general, various target properties may be validated using the proposed techniques. Preferably, these target properties should be observable in, or derivable from, the one or more images. For example, the target property may be one of a spatial distribution (e.g., where is evidence of presence of the molecule or payload in the one or more images), a spatio-temporal distribution (e.g., how does the distribution of the molecule or payload in the one or more images evolve over time), an intensity distribution (e.g., how intensive is the presence of the molecule or payload in the one or more images, e.g., over space and/or time), and a cell fate (e.g., which may be caused or influenced by the molecule).The aforementioned properties can be observed in, or derived from, the one or more images.

For example, the candidate molecules may be molecules for transporting or sequestering one or more payloads to a target region. Such molecules are useful as labels/tags, and also usually have an effect that is observable in the one or more images.

**In** some examples, the one or more payloads comprise a fluorophore. Additionally, or alternatively, the one or more payloads comprise one or more of a drug for influencing gene expression, a drug for binding as a ligand to a receptor or an enzyme, a drug acting as an allosteric regulator of an enzyme, and a drug competing for a binding site as an antagonist. Such types of payloads usually have an effect that can be observed in the one or more images.

When the target property of the candidate molecule is known, the optical imaging device, or a post-processing workflow that is applied on the images provided by the optical imaging device, can be adjusted such, that the target property can be observed in, or derived from, the respective one or more images. Accordingly, the method may comprise determining one or more imaging parameters based on the target property of the candidate molecule and obtaining the one or more images based on the determined one or more imaging parameters. These one or more imaging parameters may be used for parametrizing the optical imaging device (e.g., exposure, lighting etc.) or for post-processing the one or more images, for example.

**In** addition, an operator may be aided not only with the task of generating suitable images, but also with the task of preparing the samples such, that the target property can be observed or derived from the one or more images. For example, the method may comprise determining, for each candidate molecule, one or more parameters related to sample preparation for preparing the sample with the respective candidate molecule, and outputting the one or more parameters related to sample preparation. These one or more parameters may help the operator to perform the sample proportion, or may be used to parametrize sample preparation machinery.

In many cases, the effect a molecule has on the biological sample is not static, but rather changes over time. Therefore, multiple images, taken at multiple points in time, may be used to track the target property over time. Accordingly, the machine-learning model may be trained to process a set of images showing the biological sample at two or more points in time to output the predicted embedding of the candidate molecule. This is useful in particular with respect to the target properties "spatio-temporal distribution", "cell fate" and "intensity distribution".

As outlined above, in some cases, instead of processing the one or more images using the machine-learning model (or in addition to processing the one or more images using the machine-learning model), the one or more images may be pre-processed to derive information from the one or more images. For example, the method may comprise pre-processing, using an image processing workflow, the one or more images to generate the information derived from the one or more images. For example, the information derived from the one or more images may comprise one or more of an estimated spatial distribution of a molecule or payload, an estimated spatio-temporal distribution of a molecule or payload, an estimated intensity distribution of a molecule or payload, and a cell fate of at least one cell affected by a molecule or payload. For example, machine-learning based approaches may be used to calculate the respective distribution (e.g., using image segmentation), intensity distribution (e.g., by calculating an intensity map) or cell fate (e.g., using image classification).

In the proposed concept, one of the components being used is the machine-learning model that is used to predict the embedding of the candidate molecules. In some examples, this model can be trained (or improved) as part of the proposed method. For example, the method may comprise training the machine-learning model, using supervised learning and using a set of training data, to output the predicted embedding of the candidate molecule based on the one or more images or the information derived from the one or more images. By training the machine-learning model, a machine-learning model can be created for the specific use-case at hand.

**In** the previous examples, the focus of the method was on the validation of candidate molecules. However, in some cases, the method may also include the proposal of new candidate molecules, which are subsequently validated using the present method. For example, the method may comprise generating, using a second machine-learning model, a plurality of embeddings of molecules, and selecting the plurality of candidate molecules and corresponding embeddings from the plurality of embeddings of molecules according to a selection criterion. This may automate the pre-selection of candidate molecules which are then validated using the above examples.

To pre-select suitable candidates, different approaches may be used. For example, as outlined above, the embeddings are used to project the chemical properties of the molecules into a semantic space. It was shown that molecules that are similar to each other in the semantic space also have similar chemical properties, e.g., as a tag or label. Therefore, molecules that are similar to molecules having a desired property (e.g., that can be used as tag/label) can be pre-selected as candidate molecules based on their similarity of their embeddings. Accordingly, the method may comprise comparing the embeddings of the molecules with one or more embeddings of one or more molecules having a desired quality with respect to the target property and selecting the plurality of candidate molecules and corresponding embeddings based on the comparison.

Alternatively, or additionally, the second machine-learning model might not only output the embeddings of the molecules, but also an output indicating a quality of the molecule with respect to the target property, which can be used to pre-select the candidate molecules that have a desired quality. Accordingly, the second machine-learning model may have an output indicating a quality of the molecule with respect to the target property. For example, the selection of the plurality of candidate molecules and corresponding embeddings may be based on the output indicating the quality of the molecule with respect to the target property. For example, the output indicating the quality of the molecule may be an output of a classifier or regressor included in the second machine-learning model. In this case, a comparison with the target quality, as exhibited by known molecules, can be omitted, which may reduce the effort for pre-selecting the candidate molecules. In addition, candidate molecules that are dissimilar from previously known molecules with desired qualities might be identified.

In some cases, the second machine-learning model might not only be used to generate embeddings, but also for generating molecule embeddings de novo. For example, the plurality of embeddings may be generated autoregressively, by using the second machine-learning model to select, based on a starter token representing a portion of a molecule, one or more additional tokens representing one or more additional portions of the molecule, and generating the respective embeddings by combining the respective starter tokens with the corresponding one or more additional tokens. Thus, the second machine-learning model may be used to "auto-complete" the molecules (or rather the tokens making up the molecules), similar to the techniques used for text generation from large language models.

For example, the second machine-learning model may be a large language model (LLM) being trained on a corpus of tokenized representations of different molecules. The (large) language model may be trained using a denoising target, and/or the (large) language model may be trained to predict one or more additional tokens given one or more starter tokens. Thus, the molecules (or rather the tokens making up the molecules) may be generated using a technique that is similar to the techniques used for text generation from large language models. For example, the second machine-learning model may be trained this way as part of the proposed method. In other words, the method may comprise training the second machine-learning model using the corpus of tokenized representations of different molecules, with the training being performed using the denoising target and/or with the second machine-learning model being trained to predict the one or more additional tokens given the one or more starter tokens. This may yield a (second) machine-learning model that can be used to generate arbitrary, yet feasible, molecules based on the corpus of known molecules.

As outlined above, the second machine-learning model may (also) be used for generating the embeddings from another representation of the molecule, such as the tokenized representation of the molecule. For example, the second machine-learning model may be trained to output an embedding of a molecule based on an input comprising a representation of at least a portion of the molecule.

Some aspects of the present disclosure not covered by the scope of the invention relate to a (second) method for training a machine-learning model. The (second) method comprising obtaining a set of training data. The set of training data comprises a plurality of sets of training samples. Each training sample comprises, as training input data, a) one or more images showing a visual representation of a target property exhibited by a candidate molecule in a biological sample or b) information derived from the one or more images, and, as desired training output, an embedding of the molecule. The (second) method comprises training the machine-learning model, using supervised learning and using the set of training data, to output a predicted embedding of the candidate molecule based on the one or more images or the information derived from the one or more images. As already outlined in connection with the above (first) method, by training the machine-learning model, a machine-learning model can be created for the specific use-case at hand, to help with the validation and selection of candidate molecules.

For example, the (second) method may comprise generating at least a portion of the set of training data. For example, at least a portion of the set of training data may be generated by generating sets of one or more images showing a visual representation of a target property exhibited by a molecule in a biological sample and generating corresponding embeddings for the molecules. For example, the set of training data may comprise the sets of one or more images or information derived from the sets of one or more images and the corresponding embeddings for the molecules. This may help training the machine-learning model with molecules that are known to have desired qualities, which may improve the precision of the machine-learning model with respect to similar molecules.

Alternatively, or additionally, the(second) method may comprise generating at least a portion of the set of training data by obtaining sets of one or more images showing a visual representation of a target property exhibited by a molecule in a biological sample from a biological database, and generating corresponding embeddings for the molecules. For example, the set of training data may comprise the sets of one or more images or information derived from the sets of one or more images and the corresponding embeddings for the molecules. This may help automatically generating training data that covers a wide range of molecules.

Alternatively, or additionally, the (second) method may comprise generating a portion of the set of training data by generating, using a generative machine-learning model, sets of one or more generated images showing a visual representation of a target property exhibited by a molecule in a biological sample, and generating corresponding embeddings for the molecules. For example, the set of training data may comprise the sets of one or more generated images or information derived from the sets of one or more generated images and the corresponding embeddings for the molecules. For example, this portion of the set of training data may be generated using data augmentation techniques.

Another aspect of the present disclosure relates to a system comprising one or more processors and one or more storage devices. The system is configured to perform the method for processing images of an optical imaging device.

An aspect of the present disclosure relates to computer program with a program code for performing the method for processing images of an optical imaging device when the computer program is run on a processor.

### Short description of the Figures

Some examples of apparatuses and/or methods will be described in the following by way of example only, and with reference to the accompanying figures, in which:
- Figs. 1a and 1b: show flow charts of examples of a method for processing images of an optical imaging device;
- Fig. 2: shows an overall flow of an assay for validation of candidate molecules, such as tag candidates, with optional fine-tuning;
- Fig. 3: shows a flow chart of an example of a method for training a machine-learning model;
- Fig. 4: shows a flow chart of an example of a training of an image recognition model for automated validation of tag candidates in an assay;
- Fig. 5: shows a flow chart of an example of a method for selecting candidate molecules;
- Fig. 6: shows a flow chart of an example of a flow for training a large language model on a corpus with sequence data;
- Fig. 7: shows a flow chart of an example of a flow for autoregressive de-novo generation of new tag sequences with selection of suitable candidates in silico;
- Fig. 8: shows a flow chart of an example of a flow for fine-tuning before autoregressive de novo generation using a classification target or a regression target;
- Fig. 9: shows a flow chart of an example of a flow for scoring candidate molecules, such as tag candidates, in a latent embedding space;
- Fig. 10: shows a schematic diagram of an example of a system for performing one of the methods discussed herein; and
- Fig. 11: shows a schematic diagram of an example of a system comprising an optical imaging device and a computer system.

### Detailed Description

Various examples will now be described more fully with reference to the accompanying drawings in which some examples are illustrated. In the figures, the thicknesses of lines, layers and/or regions may be exaggerated for clarity.

In the following, some definitions are given, which can be applied to the concept discussed in the present disclosure.

A tag, as used herein, can be any kind of type protein/polypeptide, oligo-/polynucleotide, chemical (including carbohydrates, lipids) or drug.

A locus can be defined as follows: Tags can localize to multiprotein complexes, cell organelles, cells, organoids, tissues, and organs. There often is a part-of relationship between the former terms in the order of appearance. The term locus (L) can be used as an umbrella term for the multiprotein complex, cell organelle, cell, organoid, tissue, or organ the tag localizes to.

A payload Y can be defined as follows: A (primary) role of the tag in the context of the present disclosure is to carry a fluorophore or a sequence which can be labeled by fluorophore through chemical binding or association (i.e., covalent bond, coordinative bond, electrostatic interaction, nucleotide hybrization or protein-protein and protein-ligand association). In addition to visualizing a locus, the tag can be bound to, or associated with, a molecule that has an alternative function, such as acting as a drug, a regulator, inhibitor, activator, influence gene expression or alter another molecule's binding properties. That alternative molecule is called payload. It can be covalently bound to or associated with the tag (thus it can be one and the same sequence predicted by the invention).

A sequence, as used herein, is a representation of a polypeptide, polynucleotide or chemical. Polypeptides are determined by their primary structure, the amino acid sequence. Likewise, polynucleotides are represented as sequences of nucleotides. In case of chemicals, there exist IUPAC (International Union of Pure and Applied Chemistry )-conforming textual representations such as SMILES, see D. Weininger, "SMILES, a chemical language and information system. 1. Introduction to methodology and encoding rules," J. Chem. Inf. Comput. Sci., vol. 28, no. 1, pp. 31-36, Feb. 1988, doi: 10.1021/ci00057a005. Using such representations, the molecules (e.g., the tags or payloads) can be represented as a character string, so dense representations can be generated/found using large language models.

A cell fate (F) can be defined as follows: Cells can proliferate (i.e., undergo mitosis) or die (undergo necrosis in a tissue or apoptosis (controlled cell death) or unspecified cell death on the cell level). Other cell fates include migratory and static. The cell fate may be derived from time-resolved intensity distributions D(r) by identifying objects of interest in D(r), where each object instance is known, and/or by following individual objects as in single particle tracking (objects can move, stay static, split, merge), and/or by (optionally) performing a statistical analysis of the trajectories and movement patterns (e.g., free diffusion, directed motion, constrained motion) as well as ensemble statistics.

A distribution D(r) is a spatio-temporal map of intensities which are proportional to molecule concentrations in the imaged medium (cell, organelle, tissue). The vector r concatenates three spatial dimensions with time for the sake of simplicity. D(r) thus represents a probability map to encounter a particular entity E in location x at time t ("at r"), which is proportional to the concentration of *E* at *x. x* is a coordinate in *Rⁿ*, *n* ∈ {2,3}, where n can comprise 2 to 3 spatial coordinates. D can depend on additional dimensions available to the imaging device, such as channel (emission wavelength or window) coordinate, an excitation wavelength, fluorescence lifetime and derivative values, a vibrational spectral property (e.g., wavenumber) as well as other properties which can be spatially resolved, such as multi-photon excited fluorescence, second or third harmonics, polarization or other physical properties of electromagnetic radiation which are recorded by an imaging device.

Any observable, such as a distribution *D* can depend on joint conditions of one or more conditions, one or more relations *R,* the presence of one or more entities *E*. So, there can be cases such as *P*(*Dₚ* | (*E*₁; *R; E*₂, *K*₁, *K*₂, *..., Kₙ*) where one observes a distribution of e.g., a protein *E*₂ given that a chemical *E*₁ is in a relation with *E*₂ (such as being an activator thereof) and particular culture conditions *K*_{1,...,*n*} (such as temperature, presence of *E*₁ in the culture vessel at a particular concentration etc.).

A DNN is a deep neural network, which can involve any algorithm, such as MLP (MultiLayer Perceptron), CNN (Convolutional Neural Network), RNN (Recurrent Neural Network), or Transformer (a neural network mainly based on attention mechanism).

Fine-tuning is a form of training a deep learning model during which only some model parameters are varied while others remain fixed or get trained using a much (orders of magnitude) lower learning rate.

An image is a digital image, for example with dimensions XY (i.e., two lateral dimensions X and Y), XYZ (i.e., a depth-dimension Z in addition to the two lateral dimensions X+Y), XY+T (XY + Time), XYZ+C (XYZ + Channel), XYZ+T (XYZ + Time), XYZCT (XYZ + Channel + Time), XYZCT+other modalities. In other words, a 2D or nD digital image (tensor) with n EN.

Various examples of the present disclosure relate to a concept (e.g., a method, a system and/or a computer program) for generating chemicals or macromolecules which specifically target subcellular organelles in cells, organoids, or cells in tissues.

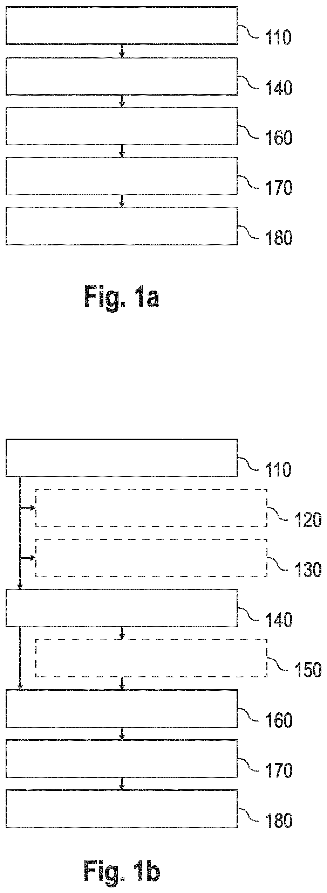

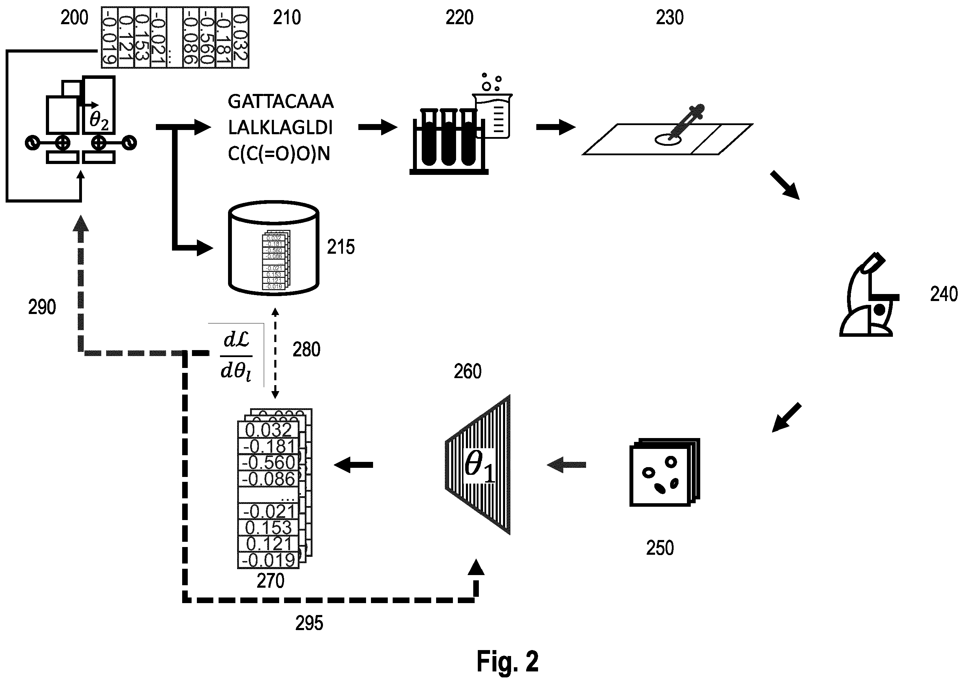

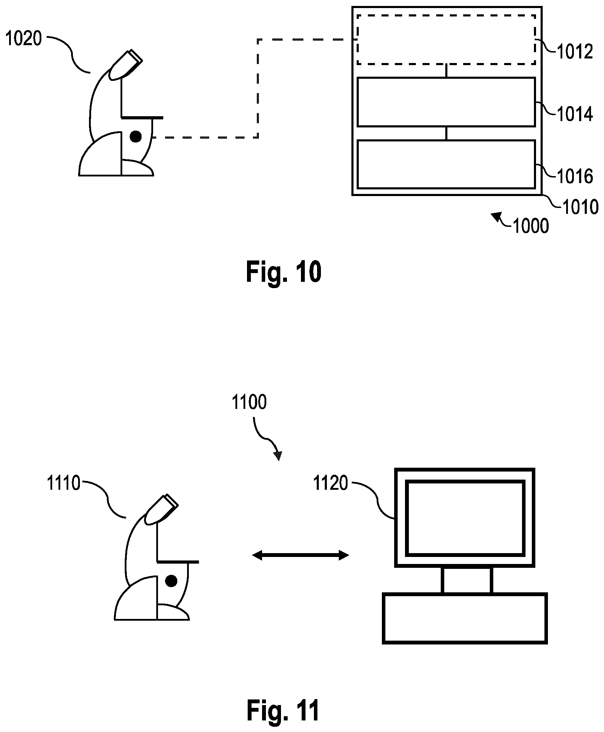

Figs. 1a and 1b show flow charts of examples of a method for processing images of an optical imaging device 240 (as shown in Fig. 2). The method comprises obtaining 110 embeddings 215 (shown in Fig. 2) of a plurality of candidate molecules. The method comprises obtaining 140, for each candidate molecule, one or more images 250 (shown in Fig. 2) of the optical imaging device. The one or more images show a visual representation of a target property exhibited by the candidate molecule in a biological sample 230 (shown in Fig. 2). The method comprises processing 160, using a machine-learning model, for each candidate molecule, the one or more images and/or information derived from the one or more images to generate a predicted embedding 270 (shown in Fig. 2) of the candidate molecule. The machine-learning model is trained to output the predicted embedding for an input comprising the one or more images and/or the information derived from the one or more images. The method comprises comparing 170 the embeddings 215 of the candidate molecules with the predicted embeddings 270 of the candidate molecules and selecting 180 one or more candidate molecules based on the comparison. For example, the method may be performed by a computer system, e.g., by the system 1010 introduced in connection with Fig. 10 and/or by the computer system 1120 introduced in connection with Fig. 11.

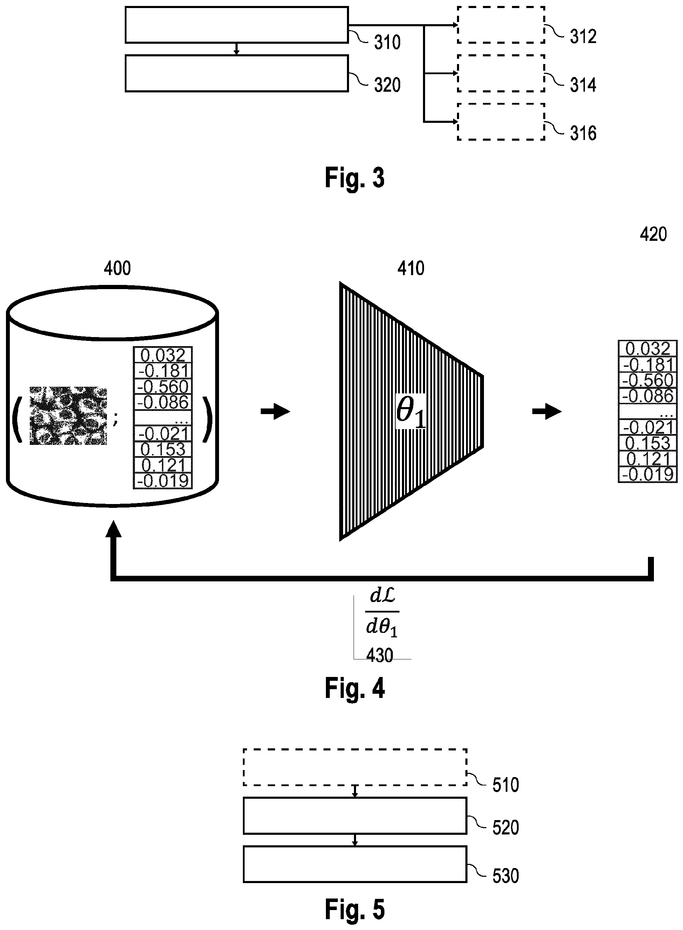

The present disclosure relates to biomedical imaging, and in particular to the validation and design of chemical tags. In this context, tags are chemicals or macromolecules (such as polypeptides, polynucleotides) which specifically target a cell organelle or another biochemically definable structure. The proposed concept relates both to the validation of such tags (which is discussed in connection with Figs. 1a to 4), and to the design of such tags (which is discussed primarily in connection with Figs. 5 to 9). While, in the following, reference is made to the design of such tags, the design of the tags is considered to be an optional aspect with respect to the method of Figs. 1a and 1b. The design of such tags is discussed in connection with Figs. 5 to 9. In some examples, both the design and validation of the tags is part of the method. In this case, one or more operations of the method of Fig. 5 may be included in the method of Figs. 1a and/or 1b. In some examples, even training of a (second) machine-learning model being used to generate the tags may be part of the method if Figs. 1a and/or 1b.

In some examples, the training of the machine-learning model (being used to predict the embeddings) may be part of the method of Figs. 1a and/or 1b. In this case, one or more operations of the method of Fig. 3 may be included in the method of Figs. 1a and/or 1b.

In the following, all of the above aspects are discussed in an interrelated manner. However, both the generation of the candidate molecules (and embeddings thereof) and the training of the machine-learning model (and of a second machine-learning model) are optional with respect to the method of Figs. 1a and/or 1b.

The proposed concept, as illustrated in connection with Figs. 1a to 2, is based on the finding, that (chemical) molecules can be represented, as words, in a semantic space, such as a semantic protein space or nucleotide space or chemical space. A semantic space is a mathematical construct used to represent the meaning of words by measuring their relationships with one another. It maps words onto a space where each word is represented by a vector that captures its meaning based on its similarity or relatedness to other words in the space. Semantic spaces are commonly used in natural language processing and computational linguistics to analyze and understand the meaning of words and to perform tasks such as text classification, information retrieval, and machine translation. In the present case, the semantic space is not used for words in the usual sense, but for string representations of the respective molecules. For example, the aforementioned string representation of a molecule may be based on the SMILE notation of molecules. The SMILE notation of molecules, which stands for "Simplified Molecular Input Line Entry System," is a way of representing the structure of a molecule using a string of characters. Each character in the string represents an atom or a bond in the molecule. The SMILE notation is often used in chemical databases and software applications to represent and search for molecular structures.

In the present context, the semantic space can be a semantic protein space, a semantic nucleotide space or a semantic chemical space, wherein molecules are represented in the respective semantic space, based on the string representations of the respective molecule. To project the respective molecules into the semantic space, embeddings are created from the string representations of the respective molecules. For example, the molecules may be taken part, either on a chemical level (by subdividing the chemical into individual nucleotides / amino acids and residue chemicals) or on a string level (by subdividing the string representations of the molecules into substrings, e.g., using byte-pair encoding or wordpiece tokenization), and encoded as tokens. For each token, a corresponding embedding in the semantic space may be calculated, e.g., using the second machine-learning model, which is introduced in more detail in the following. To obtain the embedding of the molecule, the embeddings of the tokens may be pooled, e.g., using global maximum pooling or global average pooling.

In the proposed concept, the aforementioned embeddings are used for various purposes. With respect to the method of Figs. 1a and 1b, the embeddings are used for a comparison loop, in which an embedding is predicted based on the effect a candidate molecule has on a biological sample, e.g., with respect to locus or cell fate. Care may be taken to ensure that the embedding space, i.e., the semantic space, is continuous, so that molecules with similar properties correspond to embeddings in close proximity. Continuity is encouraged by tokenization of the string representations using methods such as BPE (Byte-Pair-Encoding) and wordpiece, which are designed that way. It is also encouraged by masked language modeling, which promotes learning context (for classification) by passing outputs through a linear (dense) layer and softmax non-linearity, which treats the prediction as a probability distribution.

The method of Figs. 1a and 1b starts by obtaining 110 embeddings of the plurality of candidate molecules. In a simple implementation, the embeddings may be obtained by reading pre-defined embeddings of the candidate molecules from a memory or storage, or by calculating the embeddings from a string representation of the candidate molecules. For example, a large language model (LLM), which corresponds to the aforementioned second machine-learning model, may be used for calculating the embeddings of the string representations of the candidate molecules, e.g., using the above-mentioned tokenization. For example, as will be discussed in more detail in connection with Figs. 5 to 9, an output of a last hidden layer of a transformer block of the large language model (LLM) may be used as embedding when the LLM is supplied with the tokenized representation of the molecule. Accordingly, the second machine-learning model may be trained to output an embedding of a molecule based on an input comprising a representation of at least a portion of the molecule, e.g., based on an input comprising one or more tokens of the tokenized representation of the molecule.

However, in some examples, the proposed concept is not limited to the calculation of embeddings of a pre-defined set of candidate molecules, but to the (pre-) selection of the candidate molecules themselves. In particular, some examples of the present disclosure relate to a procedure and embodiment which uses a number of deep learning models to accelerate the screening of large candidate libraries for fluorescent tags largely *in silico* (i.e., computationally, in contrast to in *vivo*/*in vitro,* which means experimentally). The latter aspects of the method of Figs. 1a and 1b, i.e., the validation of the candidate molecules, may then be applied to candidates that are generated using the above-mentioned LLM. Using the method of Figs. 1a and 1b, an imaging device, such as a microscope, may be used to test a number of *in silico* validated candidate molecules, which are, in the following, also denoted tag candidates, as will become evident in the following.

To discuss the generation of candidate molecules that are to be included in the plurality of candidate molecules, a short excursion is made with respect to the type of molecules that are at the heart of various examples of the present disclosure. As outlined above, various examples of the present disclosure relate to the design and validation of tags. Tags are chemicals or macromolecules (such as polypeptides, polynucleotides) which specifically target a cell organelle or another biochemically definable structure. Such tags may be used for various purposes. In various examples, tags are sought that can be used to affect a target outcome at a target location of the sample. For example, a first application may be to couple these tags to fluorophores as a contrasting agent in light microcopy. A second potential application is to couple the tag to a chemical which can act as a drug or drug delivery system. In both cases, the tag is used to transport or sequester a payload, such as the fluorophore or drug, to a target location of a sample. Accordingly, the candidate molecules may be molecules for transporting or sequestering one or more payloads to a target region. As outlined above, the one or more payloads may comprise a fluorophore. In addition, or alternatively, the one or more payloads may comprise one or more of a drug for influencing gene expression, a drug for binding as a ligand to a receptor or an enzyme, a drug acting as an allosteric regulator of an enzyme, and a drug competing for a binding site as an antagonist.

Various examples of the present disclosure can produce "tags" which have either of or a combination of the following properties. For example, the tags may comprise one or more low molecular weight chemicals or fluorophores which selectively or specifically bind to target structures in the specimen directly. Alternatively, or additionally, the tags may comprise one or more low molecular weight fluorophores which can be covalently bound to a delivery system such as antibodies or FISH probes. Alternatively, or additionally, the tags may comprise at least one polypeptide which folds into a fluorescent protein and can be engineered to be expressed in a live specimen. Alternatively, or additionally, the tags may comprise one or more low to mid molecular weight fluorophores which specifically binds to a peptide or nucleotide within the cell. Both, the fluorophore as well as the target sequence can be predicted and tested using the proposed concept. Alternatively, or additionally, the tags may comprise one or more adaptor molecules, whose sole purpose is to transport and specifically attach a fluorophore or another molecule which can act as a drug to a target in the specimen. It is evident, that all of the above tags fit the above description of transporting or sequestering a payload, such as a fluorophore or a drug, to a target region.

Therefore, an aspect of the proposed concept can be used to predict suitable tags which transport (or sequester) a fluorophore to a target region, a specific locus where they can be detected by a bioimaging device (e.g., fluorescent light microscope). In this sense a tag can be also viewed as a shuttle transporting fluorophores or other "payloads" to a target. The tag / shuttle is effectively a chemical adaptor between cellular target molecules and the fluorophore / payload. Due to its structure, a cell will target it to a particular compartment (e.g., using protein targeting). A secondary goal of this aspect of the proposed concept can be to (optionally ) transport a molecule (payload) alternatively or additionally to the fluorophore. This secondary payload can act as a drug, that is it can influence gene expression (e.g., as upregulator, downregulator), bind as a ligand to a receptor or an enzyme, act as an allosteric regulator of an enzyme, or compete for a binding site as an antagonist. The main idea behind the secondary payload is to manipulate living cells, organoids, or tissues in a biological experiment to study metabolism, signal transduction, cell biology or neurobiology using a bioimaging system (e.g., microscope). So, the payload may be expected to act as a research tool which can be added to a cell culture or other specimen as a commercially available consumable. In principle, the concept may be applied to pharmaceutical applications, too.

The goal in finding suitable tags is the identification of molecules that affect a desired outcome at a target region. Therefore, the suitability of a molecule for use as a tag is dependent on whether the tag has the desired outcome at the target region. This enables the methodology employed herein, in which images are used to validate the suitability of the tags. By taking images of the biological sample, information can be collected on how the molecule affects the biological sample. In particular, the images may show how the molecule and/or the payload is distributed across the biological sample, or what effects are caused by the distribution of the molecule or payload, with respect to cell fate. Accordingly, the target property being observed herein may be one of a spatial distribution (e.g., where is evidence of presence of the molecule or payload in the one or more images), a spatio-temporal distribution (e.g., how does the distribution of the molecule or payload in the one or more images evolve over time), an intensity distribution (e.g., how intensive is the presence of the molecule or payload in the one or more images, e.g., over space and/or time), and a cell fate (e.g., which may be caused or influenced by the molecule). The target property can both be used to validate the candidate molecules, and to pre-select candidate molecules that may have a desired quality.

To generate the plurality of candidate modules, the candidate molecules (e.g., the "tags" may be predicted using a Large Language Model. A Large Language Model (LLM) is a type of artificial intelligence model used for natural language processing tasks such as text generation, machine translation, sentiment analysis, and more. LLMs are designed to mimic human language patterns and are trained on massive amounts of language data, typically in the form of text. They can generate coherent, contextually appropriate sentences, and often produce outputs that are indistinguishable from those generated by humans. Examples of popular LLMs include GPT-3 (Generative Pre-trained Transformer 3) and BERT (Bidirectional Encoder Representations from Transformers). In the present case, as outlined above, the Large Language Model is not used for the task of natural language processing or generation, but rather for the task of computing embeddings from string representations of molecules, and also for the purpose of generating valid molecules (as sequences and embeddings), which can potentially be used as candidate molecules.

To generate the candidate molecules, or rather molecules, of which some can be included in the plurality of molecules, the aforementioned LLM may be used. For example, the LLM may be pre-trained to created dense embeddings of nucleotide sequences, protein sequences, text representations of chemicals (such as SMILE, SELFIE) or graph representations of molecules. For example, the second machine-learning model may be a large language model being trained on a corpus of tokenized representations of different molecules. For example, the second machine-learning model may be trained to output, a sequence representing the molecule (e.g., as a string or as sequence of tokens representing the candidate molecule) and the embedding of the molecule. In some cases, the second machine-learning model may be trained as part of the method. Accordingly, in some examples, the method may comprise training 510 the second machine-learning model using a corpus of tokenized representations of different molecules. In particular, the language model may be trained using a denoising target, and/or the language model may be trained to predict one or more additional tokens given one or more starter tokens. Examples with respect to the training of the LLM, and selection of suitable candidate molecules, are given in connection with Figs. 5 to 9. As alternative to training the LLM, an off-the-shelf LLM model being trained to create the dense embeddings of the nucleotide sequences, protein sequences, text representations of chemicals or graph representations of molecules may be used, or the training may be performed by a different entity than the entity performing the presently-discussed method.

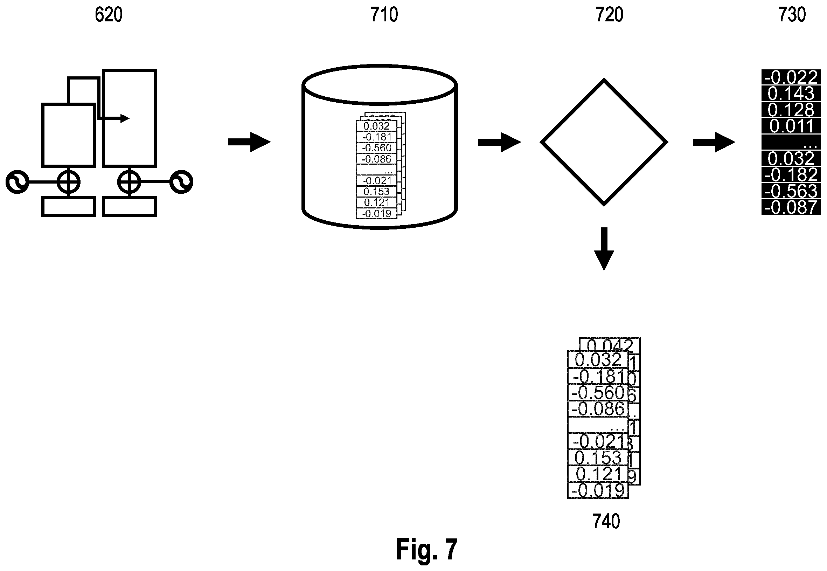

This LLM may then be used for autoregressive de novo synthesis of tag candidates (optionally after fine-tuning to a locus prediction objective, which is illustrated in connection with Fig. 7). Accordingly, as shown in Fig. 5, the method may comprise generating 520, using a second machine-learning model, a plurality of embeddings of molecules. The output of the model may be stored, with the output comprising a sequence as well as the activations output by the last hidden layer of the transformer block of the model, which is the model's hidden representation as a latent vector of the tag candidate. This latent vector is also referred to as (molecule) embedding in the context of the present disclosure.

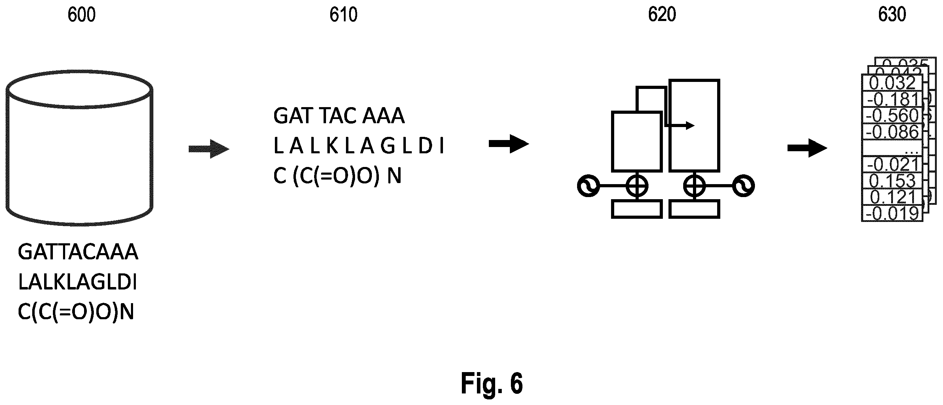

The generation of the candidate molecules (and their embeddings) is detailed in connection with Figs. 6 and 7, for example. As a short summary, the candidate molecules (and in particular the embeddings of the candidate molecules) may be generated autoregressively by feeding the second machine-learning model a starter token (representing a portion of the molecule) and letting the second machine-learning model iteratively pick additional tokens to be combined with the starter token until a stopping condition is met. In other words, the plurality of embeddings may be generated autoregressively, by using the second machine-learning model to select, based on a starter token representing a portion of a molecule, one or more additional tokens representing one or more additional portions of the molecule, and generating the respective embeddings by combining the respective starter tokens with the corresponding one or more additional tokens. In effect, the process is similar to the generation of text by BERT-like LLMs.

As further shown in Fig. 5, the method may comprise (pre-)selecting 530 the plurality of candidate molecules and corresponding embeddings from the plurality of embeddings of molecules according to a selection criterion. To be clear - the selection 530 of the plurality of candidate molecules according to the selection criterion is different from the selection 580 of the one or more candidate molecules based on the comparison of the embeddings of the molecules with the predicted embeddings. The (pre-)selection being performed at this stage is used to (pre-)select candidate molecules that are to be tested in a biological sample.

To perform the (pre-)selection, one or both of the following approaches may be used. In a first approach, the generated molecule embeddings are compared with embeddings of molecules with known desirable qualities. Using the output of the model (i.e., the embeddings, and thus location in semantic space), a distance between the tag candidate's embedding (i.e., the embedding of the candidate molecule) and the embedding of a known dye with known biological properties, such as locus, may be calculated. Using distance metrics, one can effectively test tag candidates for desired properties *in silico.* Accordingly, the method may comprise comparing the embeddings of the molecules with one or more embeddings of one or more molecules having a desired quality with respect to the target property and selecting the plurality of candidate molecules and corresponding embeddings based on the comparison.

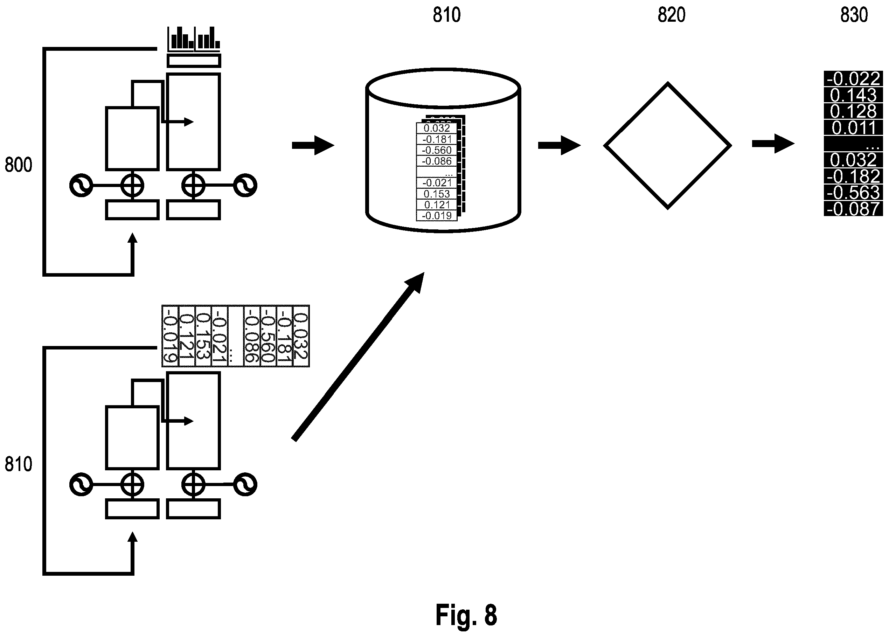

In a second approach, which is illustrated in connection with Fig. 8, the second machine-learning model may be trained to output, in addition to the sequence (i.e., a string or token representation of the candidate molecule) and the embedding, an output indicating a quality of the molecule with respect to the target property. In other words, the second machine-learning model may have an output indicating a quality of the molecule with respect to the target property. For example, the output indicating the quality of the molecule may be an output of a classifier or regressor included in the second machine-learning model. In this case, the selection of the plurality of candidate molecules and corresponding embeddings may be based on the output indicating the quality of the molecule with respect to the target property. For example, molecules may be selected for the plurality of candidate molecules if the output indicating the quality of the molecule with respect to the target property indicates that the molecule exhibits a desired quality with respect to the target property.

Up to this point, this aspect of the proposed concept has produced likely tag candidates (i.e., molecule candidates) with desired properties (i.e., with a desired quality), thus narrowing down the search space for costly chemical synthesis and microscopic validation. In order to automate the validation further, a training corpus of pairs of images with matching tag embeddings may be created to train a visual model (i.e., the machine-learning model). Such a visual model can be used to a) validate microscopic pictures to score successful locus (or other visible properties/qualities) of a tag candidate, b) search a repository of images for spatio-temporal distributions with desired properties (e.g., to find existing molecules/tags), and/or c) act as a perceptual loss function in training the deep learning models being used in the proposed concept in an end-to-end fashion across prediction, synthesis, and microscopy of tag candidates. Various examples of the present disclosure thus relate to a system that employs large language models and image generation by deep learning in conjunction with a biomedical imaging device to design chemical tags. The training of such a machine-learning model is discussed in connection with Figs. 3 and 4.

In the method of Figs. 1a and/or 1b, such a machine-learning model is used for purpose a), to validate microscopic pictures to score successful locus (or other visible properties/qualities) of a tag candidate. For this purpose, the method comprises obtaining 140, for each candidate molecule, one or more images 250 of the optical imaging device, with the one or more images showing a visual representation of the target property (i.e., the a spatial distribution, spatio-temporal distribution, intensity distribution, and cell fate) exhibited by the candidate molecule in a biological sample 230. For example, the one or more images 250 may be obtained from an optical imaging sensor of a microscope, e.g., the microscope 1020 shown in Fig. 10. For example, the one or more images may comprise images taken using different imaging modes (e.g., white light imaging and fluorescence imaging in one or more wavelength bands), or using different illumination modes (e.g., white light illumination and fluorescence excitation illumination). For example, the one or more images (or rather two or more images) may be taken at two or more different points of time (of an experiment). Additionally, or alternatively, the one or more images may be taken by different optical imaging sensors of the optical imaging device.

In some cases, the one or more images are processed, as they are, by the machine-learning model. In some cases, however, some amount of pre-processing may be applied to the one or more images, and the result of the pre-processing may be processed by the machine-learning model. Accordingly, as further shown in Fig. 1b, the method may comprise pre-processing 150, using an image processing workflow, the one or more images to generate the information derived from the one or more images. For example, the image processing workflow may include both one or more deterministic image processing steps (such as contrast enhancements, wavelength band filtering etc.) and one or more machine-learning based image analysis steps (such as image segmentation, object detection, regression and classification). In particular, the pre-processing of the one or more images may be performed to determine an estimate of the respective target property (e.g., using a machine-learning based image analysis step, such as image segmentation or object detection to identify the location/spread of the molecule or payload in the one or more images, or classification to classify the cell fate). For example, the pre-processing may be used to determine one or more of an estimated spatial distribution of a molecule or payload, an estimated spatio-temporal distribution of a molecule or payload, an estimated intensity distribution of a molecule or payload, and a cell fate of at least one cell affected by a molecule or payload. Accordingly, the information derived from the one or more images may comprise one or more of the estimated spatial distribution of a molecule or payload, the estimated spatio-temporal distribution of a molecule or payload, the estimated intensity distribution of a molecule or payload, and the cell fate of at least one cell affected by a molecule or payload. For example, the information derived from the one or more images may be provided as image or images, or as two-dimensional map with values derived from the one or more images. For example, in the case of the information derived from the one or more images comprising the estimated spatial distribution, the estimated spatial distribution may be provided as monochrome image, with the color (black or white) of each pixel indicating, whether the payload or molecule is detected at a corresponding pixel of an image of the one or more images. In the case of the information derived from the one or more images comprising the estimated spatio-temporal distribution, the estimated spatial distribution may be provided as series of monochrome images, with each monochrome image representing the spatial distribution at one of two or more points in time during the experiment. In the case of the information derived from the one or more images comprising the intensity distribution, a visual representation of the intensity distribution in the resulting image, e.g., a position on a greyscale or color gradient of a resulting single-channel/greyscale image, may reflect the number of molecules or payload detected at a pixel of the one or more images. In case the information derived from the one or more images is provided as a map, the values indicated by the map may be selected similar to the image, e.g., 0 and 1 instead of a monochrome image, and a range of values, e.g., 0 to 255, in case of the intensity distribution. In the context of the present application, such information derived from the one or more images are also referred to as intensity distribution D(r) (in the case of intensity distribution, which may be a spatio-temporal intensity distribution), or as cell fate F.

To aid in the analysis of the target property, the image processing workflow, and/or the image acquisition by the optical imaging sensor, may be adjusted according to the target property being analyzed. For example, as further shown, the method may comprise determining 120 one or more imaging parameters based on the target property of the candidate molecule, and obtaining the one or more images based on the determined one or more imaging parameters. For example, depending on the target property, different illumination wavelength bands, different wavelength band filtering parameters, different image analysis models etc. may be used.

The machine-learning model is trained to output the predicted embedding for an input comprising the one or more images and/or the information derived from the one or more images. Details with respect to the training are given in connection with Figs. 3 and 4. As a short summary, a supervised learning-based training may be used, to train the machine-learning model to output an embedding for an input comprising the one or more images or the information derived from the one or more images. As the training of the machine-learning model can be conducted as part of the present method, the method may comprise training 320 the machine-learning model, using supervised learning and using a set of training data, to output the predicted embedding of the candidate molecule based on the one or more images or the information derived from the one or more images. In particular, as the spread of the molecule or payload over time is of interest, two or more images (or two or more sets of information each derived from an image of the two or more images) taken over two or more points of time during the experiment may be input into the machine-learning model. Accordingly, the machine-learning model may be trained to process a set of images showing the biological sample at two or more points in time (or sets of information derived from the set of images) to output the predicted embedding of the candidate molecule. For example, in some cases, the set of images (or two or more sets of information) may be input into the machine-learning model via two or more separate inputs of the machine-learning model. Alternatively, the machine-learning model may include a "memory", such as a Long Short-Time Memory, LSTM, to "remember" the images or information derived from the images. In this case, a single input may suffice.

The machine-learning model is used to process 160 the one or more images and/or information derived from the one or more images to generate the predicted embedding of the candidate molecule. In other words, based on the one or more images, the machine-learning model predicts, what/which molecule has caused the effect (with respect to the target property) to the biological sample, as point in the semantic space (i.e., as predicted embedding). The resulting predicted embeddings are then compared 170 to the embeddings of the candidate molecules, to determine whether the candidate molecules 170 have the expected effect with respect to the target property. For example, for a candidate molecule, the embedding predicted based on the one or more images showing the visual representation of the target property exhibited by the candidate molecule in a biological sample is similar to the embedding calculated (using the second machine-learning model) for the candidate molecule, the candidate molecule may be selected 180 for the one or more candidate molecules, as the candidate molecule has behaved as expected. If, however, for a candidate molecule, the embedding predicted based on the one or more images showing the visual representation of the target property exhibited by the candidate molecule in a biological sample is dissimilar to the embedding calculated (using the second machine-learning model) for the candidate molecule, the candidate molecule might not be selected 180 for the one or more candidate molecules, as it has not behaved as expected.

In the following, in Fig. 2, an overall flow is shown, from de-novo generation of candidate molecule embeddings to the comparison 280 of the predicted embeddings and the embeddings calculated for the de-novo generated candidate molecules. It is to be noted that operations 215, 250, 260 and 280 are at the core of the method of Figs. 1a and/or 1b. Additional operations, such as the generation of candidate molecule embeddings using a pre-trained LLM 200, chemical synthesis 220, sample preparation 230 and imaging 250 are either optional or entirely outside the scope of the method of Figs. 1a and/or 1b.

More details and aspects of the method of Figs. 1a and/or 1b are mentioned in connection with the proposed concept or one or more examples described above or below (e.g., Fig. 2 to 11). The method may comprise one or more additional optional features corresponding to one or more aspects of the proposed concept, or one or more examples described above or below.

Fig. 2 shows an overall flow of an assay for validation of candidate molecules, such as tag candidates, with optional fine-tuning. Fig. 2 illustrates, among other, the testing of predicted tags using an imaging device. In order to test the validity of potential tag candidates, the respective molecules may be synthesized, samples produced (e.g., stained, made transgenic) and imaged.

The pretrained large language model 200 (i.e., the second machine-learning model) predicts *de novo* tag candidates (i.e., candidate molecules) 210 and its semantic embeddings 215. Chemical synthesis 220 of the candidate molecules may be automated as much as possible, likewise sample preparation 230 of the biological sample with the respective candidate molecule. Accordingly, the method of Fig. 1b may further comprise determining 130, for each candidate molecule, one or more parameters related to sample preparation 220 for preparing the sample with the respective candidate molecule, and outputting the one or more parameters related to sample preparation. Chemical synthesis may involve operations, such as protein expression and purification in bacterial or metazoan cell systems, DNA/RNA synthesis and amplification, direct synthesis of peptides, chemical combinatorial synthesis followed by additional purification and quality control. Sample preparation may involve culturing 2D or 3D cell culture, organoids, spheroids, sectioning tissues or preparing organs (e.g., by dissection, clearing) or preparation of whole animals for *in vivo* imaging (e.g., cranial windows in mouse cerebral cortex imaging). Sample preparation may further involve production of samples in suitable vessels for microscopy as well as staining, electroporation, fixation, transfection or washing of samples in presence of the respective tag molecule.

The design of the data recording part 240 depends on the goal (i.e., the target property) of the new polymers or compounds to be found. If, for example, localization is the target (target property), then an imaging device will be used. If binding assays (as in ligand and protein) are conducted, it is more practical to use an optical test, that is any form of spectrophotometry. In the latter case, the molecule of interest may be chemically coupled to a proto-chromophore, which develops its chromogenic or fluorescent reporter properties upon chemical cleavage or an enzymatic reaction. The latter read-out can be scaled up more easily than imaging. Reporter read-outs in optical tests can involve one or more of UV light, visible light in absorption or fluorescence, infrared absorption, light polarization, optical rotatory dispersion, static and dynamic light scattering, fluorescence lifetime spectroscopy, and fluorescence correlation spectroscopy. Imaging 240 may typically involve microscopy, high content screening, and/or mesoscopy (for observing large specimen such as whole organs or organisms in vivo). Imaging modalities can comprise one or more of visible and UV (UltraViolet) light, infrared light (including vibronic microscopy such as Coherent anti-Stokes Raman Scattering, CARS, or Stimulated Raman Scattering, SRS), multiphoton excitation and other forms of non-linear microscopy, fluorescence lifetime imaging, fluorescence correlation spectroscopy, image correlation microscopy, polarized light microscopy, label-free contrasting including phase contrast, differential interference contrast, intensity modulation contrast, optical nanoscopy below the Abbe limit or combinations thereof. However, regardless of the data recordation modality, the respective information may be obtained as an image or an image-like data structure (e.g., a two-dimensional map).

Some of the operations, in particular chemical synthesis 220, sample preparation 230 and data recordation 240 are laborious, cost-intensive and to some extent require manual intervention. Therefore, it may be desired to reduce the number of tag candidates validated in vitro or in vivo by this assay. As tag candidates' embeddings are expected to localize in close proximity in semantic space, a stochastic sample of a set of tag candidates can be taken and only the stochastic sample might be validated in vitro. The other tag candidates can be assayed in silico (i.e., computationally) through replacing operations 220-240 by a generative model using a conditional generative neural network approach, as discussed in connection with Fig .4. In effect, a "digital twin" can be generated, for predicting the effect of the candidate molecule on the sample. Such a model takes a tag embedding as input and produces a likely image as out-put. The latter can be forward passed through image recognition model 260. The precision of this approach can be increased if not only one desired property, such as locus, is taken into account, but also other molecular properties, such as toxicity or biological activity or chemical properties (see also embodiment 3 for such properties). The large language model 200 can be fine-tuned in order to predict multiple such properties as shown in Fig. 8. This approach may help with reducing the number of candidates to the point that the feasibility of actual assaying in vitro / in vivo increases.

The multiple images 250 produced, using data recordation 240 or using a generative model, show observables such as spatio-temporal distribution D(r) or cell fate (F). Typically, these observables will be image stacks with the dimensions X,Y,Z,T,CH (3 spatial dimensions, time and "channel" which is a proxy for spectral emission wavelength bands). Other dimensions may include one or more of stage position (in the x- and y-dimension), logical position on sample carrier ("well", "biopsy core number", "object number"), direction of illumination for light sheet imaging, polarization, and fluorescence lifetime in continuous or gated representation. Microscopically visible spatio-temporal distribution, D(r), as a read-out for success can be complemented by additional laboratory appliances for testing physico-chemical properties of predicted and synthesized tags, such as solubility (as a function of temperature, pH and ion strength) or toxicity to cell culture, organoids or whole animals. In particular toxicity can be assessed microscopically via the ratio of live vs dead cells as a function of tag concentration in culture (i.e., the microscope specimen).

The images (and/or information derived from the images), and optionally additional read-outs discussed above, are input to the trained (image recognition) model 260 (i.e., the machine-learning model) to infer a probable tag embedding 270. The latter is expected to be in close proximity to the de novo predicted tag in embedding space. Their proximity may be measured using a distance metric. At that point, quantitative validation may be complete, and a ranking of potential candidate tags can be produced. Optionally, a differentiable loss may be computed 280, which can be used to fine-tune end-to-end all or some of the deep learning models involved in the assay, in particular the large language model (i.e., the second machine-learning model) 200 for *de novo* prediction and the (image recognition) machine-learning model 260 used for scoring. In some cases, the parameters of the machine-learning model 260 may remain fixed, and activations from one or more layers may be extracted while fine-tuning the second machine-learning model 200. In this case, the machine-learning model 260 effectively acts as a perceptual loss function for training the second machine-learning model 200. Predictions, loss function or extracted activations / hidden representations from the machine-learning model 260, but also from the second machine-learning model 200 can feed back to lab appliances and imaging devices as in chemical synthesis 220, sample preparation 230 or data recordation 240 to modify their control flow and / or modify parameters of machine learning models therein.

Not only tags (with desired locus) can be designed using the proposed concept, but also other "payload" molecules with their respective properties (such as biological or drug activity). For example, the tag molecule acts as shuttle (such as signal sequence determining locus) and / or adaptor molecule for the payload molecule to be targeted specifically. In the latter case, the proposed concept may be used for optimizing two such molecules (tag, payload) jointly. Consequently, both the second machine-learning model 200 and the machine-learning model 260 can possess not one, but two outputs, with each of which being fine-tuned by a loss function 290.

The backpropagation of loss gradients in Fig. 2 can be used to influence the control flow of laboratory appliances or imaging devices. In case the control flow of those devices is governed by a machine learning model, then the parameters of the latter can be fine-tuned as well. In such a way all components of the assay can be adapted to achieve minimal error (loss) between de novo predicted tag candidates and inferred embeddings of actual observables elicited by these tag candidates.

More details and aspects of the flow of Fig. 2 are mentioned in connection with the proposed concept or one or more examples described above or below (e.g., Fig. 1a to 1b, 3 to 11). The flow may comprise one or more additional optional features corresponding to one or more aspects of the proposed concept, or one or more examples described above or below.

Fig. 3 shows a flow chart of an example of a method for training a machine-learning model, i.e., the machine-learning model for processing the one or more images or the information derived from the one or more images, as discussed in connection with Figs. 1a to 2. The method comprises obtaining 310 a set of training data. The set of training data comprises a plurality of sets of training samples. Each training sample comprises, as training input data, at last one of a) one or more images showing a visual representation of a target property exhibited by a candidate molecule in a biological sample and b) information derived from the one or more images, and, as desired training output, an embedding of the molecule. The method comprises training 320 the machine-learning model, using supervised learning and using the set of training data, to output a predicted embedding of the candidate molecule based on the one or more images or the information derived from the one or more images. For example, the method may be performed by a computer system, e.g., by the system 1010 introduced in connection with Fig. 10 and/or by the computer system 1120 introduced in connection with Fig. 11. For example, the method may be performed by the same computer system, or by a different computer system as the method of Figs. 1a and/or 1b and the method of Fig. 5.

While Figs. 1a to 2 primarily relate to the application of the machine-learning model, Figs. 3 and 4 relate to its training. The (image recognition) machine-learning model can be generated using the following operations. In the following, the machine-learning model is also referred to as the "image recognition" machine-learning model. However, this does not necessarily indicate, that the machine-learning model performs recognition (e.g., object recognition) on image data. It merely indicates that the machine-learning model processes images or information derived from images. The term was chosen to delineate the machine-learning model from the second machine-learning model (i.e., the (large) language model).

In the following, a short introduction of machine-learning is given, with reference to the training of the machine-learning model, as discussed in connection with Figs. 3 and 4. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g., words or sentences) and associated training content information (e.g., labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model.

The provided data (e.g., sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e., each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g., a classification algorithm, a regression algorithm or a similarity learning algorithm. Classification algorithms may be used when the outputs are restricted to a limited set of values (categorical variables), i.e., the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are.

In the method of Fig. 3, supervised learning is also used to train the machine-learning model. As the output of the machine-learning model is the prediction of an embedding, i.e., a position in semantic space, the machine-learning model may be trained as a regressor, to output the embedding as number (or numbers) pointing towards the position in semantic space.

As training data for training the machine-learning model, the set of training data is used. As the machine-learning model is to be applied on the one or more images and/or the information derived from the one or more images, the training data may comprise images and/or information derived from the one or more images that are similar to the one or more images and/or information derived from the one or more images used in connection with the method of Figs. 1a and/or 1b. Thus, each training sample comprises, as training input data, at last one of a) one or more images showing a visual representation of a target property exhibited by a candidate molecule in a biological sample and b) information derived from the one or more images, with a format that is similar to the format used in connection with the method of Figs. 1a and/or 1b. For example, in some examples, the same optical imaging device may be used to obtain the images included in the set of training data, or the images being used may be processed to resemble the images output by the optical imaging system used for the method of Figs. 1a and/or 1b. Similarly, the same image processing pipeline may be used to compute/determine the information derived from the one or more images. Similarly, the embedding used as desired output has a format that is similar to the format used for the embeddings of the candidate molecules introduced in connection with Figs. 1a to 2.

First, a training corpus of pairs of images (corresponding to D(r), for example) and respective tag embeddings can be created. Once it is clear what kind of sequence S (e.g., what kind of molecule) is to be predicted, a training corpus (i.e., the training data set) can be created. The training corpus may comprise or consist of sequence embeddings of molecules with known locus (i.e., the target property, can be 1 or many loci) as label and a distribution D(r) or cell fate F as input (as one or more images or as information derived from the one or more images). These pairs are subsequently used for (supervised) training.

In the following, three approaches for gathering training samples for the set of training data are introduced, which may be used alone or in combination with each other to obtain the set of training data.

For example, the training corpus may be created using microscopy and a bank of known tags, by recording images using an imaging device, and by staining a particular structure specifically with known tags. Accordingly, the method may comprise generating 312 at least a portion of the set of training data by generating sets of one or more images showing a visual representation of a target property exhibited by a molecule in a biological sample and generating corresponding embeddings for the molecules. In this case, the set of training data may comprise the sets of one or more images or information derived from the sets of one or more images and the corresponding embeddings for the molecules. For example, the respective property, e.g., the spatio-temporal intensity distribution D(r) or cell fate F can be measured using an imaging device with a sample that shows the locus of interest. The sample may be labeled with a tag of known locus for each D(r) / F. For each pair of input and (tag) embedding, multiple instances may be recorded. Typically, the imaging device as well as laboratory appliances such as liquid and sample handling may be set up to allow for screening a large number of tags in a locus of a particular sample and record their corresponding observable (D(r) /F, i.e., the target property) automatically.

Alternatively, or additionally, one or more publicly available atlases of recorded images may be used to create (at least a part of the training corpus). Accordingly, the method may comprise generating 314 at least a portion of the set of training data by obtaining sets of one or more images showing a visual representation of a target property exhibited by a molecule in a biological sample from a biological database and generating corresponding embeddings for the molecules. In this case, the set of training data may comprise the sets of one or more images or information derived from the sets of one or more images and the corresponding embeddings for the molecules. The scientific research community in life sciences releases large bodies of data with imagery of biological specimen. Usually, these are labeled with tags of known origin and the labeling protocol is recorded in the same database. In case such publicly accessible data repositories are available, they can be used to provide ground truth. Otherwise, this publicly available data can be complemented with the images recorded by the imaging device (see above), or images generated using a conditional generative neural network (see below).

Alternatively, or alternatively, images for the training corpus can be generated as predicted intensity distributions by using a conditional generative neural network trained to use a candidate tag embedding as a condition (e.g., for semi-supervised training with synthetic data). Accordingly, the method may comprise generating 316 a portion of the set of training data by generating, using a generative machine-learning model, sets of one or more generated images showing a visual representation of a target property exhibited by a molecule in a biological sample, and generating corresponding embeddings for the molecules. In this case, the set of training data may comprise the sets of one or more generated images or information derived from the sets of one or more generated images and the corresponding embeddings for the molecules. Since screening large libraries of molecules and samples tagged therewith is costly, (part of) the training corpus may be generated using a generative neural network. Since the tag embeddings are already known or can easily be created with a trained large language model (as described in connection with Figs. 5 to 9), the expected observable may be predicted for a given tag embedding. However, a variational autoencoder does not appear feasible for creating both, image (D/F ) and tag embedding. From literature, a form of conditional generative adversarial network (cGAN) is known, which may have disadvantages, such as imprecise sampling of probability distribution, difficult training, or mode collapse. However, such a cGAN might still be used to generate a part of the corpus.

Alternatively, the images may be created with a normalized flow model (also a form of DNN), which is conditioned on a desired tag embedding and creates the expected observable D(r)/F. Such a model can be used for creating a visual representation of the expected observable in human-understandable format for exploratory data analysis, quality control and documentation, and for enhancing the training corpus for training the machine-learning model by adding additional data for semi-supervised training.

For example, the following Bayes rule may be used in this context. Bayes' rule, also known as Bayes' theorem or Bayes' law, is a mathematical formula that is used to calculate the probability of an event based on prior knowledge or information. In the present case, the posterior probability P(S|D) is the probability to be tag (embedding) given a particular image D, where S is the tag embedding and D is the intensity distribution D(r). The likelihood P(D|S) is the probability of seeing an image D given the sample is stained with tag (embedding) S. The prior knowledge P(S) is the probability to find tag (embedding) S given the whole corpus of all possible tags (i.e., chemicals).

Subsequently, the machine-learning model may be trained as image recognition deep neural network configured to predict tag embeddings when given an image of the likely distribution of this tag in cells or tissues.

Then, an imaging device may be used to test the predicted and synthesized tag in live cells, organoids or tissues or another setup suitable for imaging and confirm it localizes as depicted in the training image, as shown in connection with Figs. 1a to 2. This latter image recognition neural network may be used, when fully trained, to evaluate images of synthesized / expressed tags imaged *in vivo* / *in vitro.* This evaluation allows for automated validation or scoring of image tag candidates by applying a distance metric between the tag embedding predicted by this image recognition model and a tag embedding of with known properties as computed by a large language model (i.e., the second machine-learning model introduced in connection with Figs. 1a to 2, 5 to 9).

In addition, automation of tag candidate validation may be performed by using the trained image recognition model as a perceptual loss function to fine-tune the language model (i.e., the second machine-learning model) and possibly influence the control flow of the microscope and other laboratory automation appliances involved in synthesizing the tag candidates, sample and liquid handling as well as imaging.

More details and aspects of the method of Fig. 3 are mentioned in connection with the proposed concept or one or more examples described above or below (e.g., Fig. 1a to 2, 4 to 11). The method of Fig. 3 may comprise one or more additional optional features corresponding to one or more aspects of the proposed concept, or one or more examples described above or below.

In Fig. 4, an example of the training of the machine-learning model is given. Fig. 4 shows a flow chart of an example of a training of an (image recognition) machine-learning model for automated validation of tag candidates in an assay. The training is used to train the machine-learning model to predict tags with desired localization patterns. A training corpus 400, compiled as illustrated above, comprising pairs of images with corresponding tag embeddings is used to train the (image recognition) machine-learning model 410 with images as input and regression to the corresponding tag embedding presented at the output 420. A suitable objective function such as the L1 norm or L2 norm may be used to compute and backpropagate the loss gradient 430 for training.

A machine-learning model 410 thus trained is now configured (i.e., trained) to predict the most likely tag embedding for a given input image (of the observable D(r) or F). For example, a model thus trained may be used for validating images from an assay to test predicted tag embeddings once synthesized, applied to a specimen and imaged. The model output may then be compared to the tag embedding of the *de novo* prediction of the respective tag. This enables automated validation of newly designed tags. Additionally, or alternatively, the machine-learning model may be used for using a suitable objective function to measure the error between de novo prediction and embedding corresponding to the actual observable (e.g., locus, intensity distribution, cell fate). The error can be backpropagated end-to-end for training or fine-tuning of both or either of the image recognition model and the large language model.

More details and aspects of the flow of Fig. 4 are mentioned in connection with the proposed concept or one or more examples described above or below (e.g., Fig. 1a to 3, 5 to 12). The flow of Fig. 4 may comprise one or more additional optional features corresponding to one or more aspects of the proposed concept, or one or more examples described above or below.

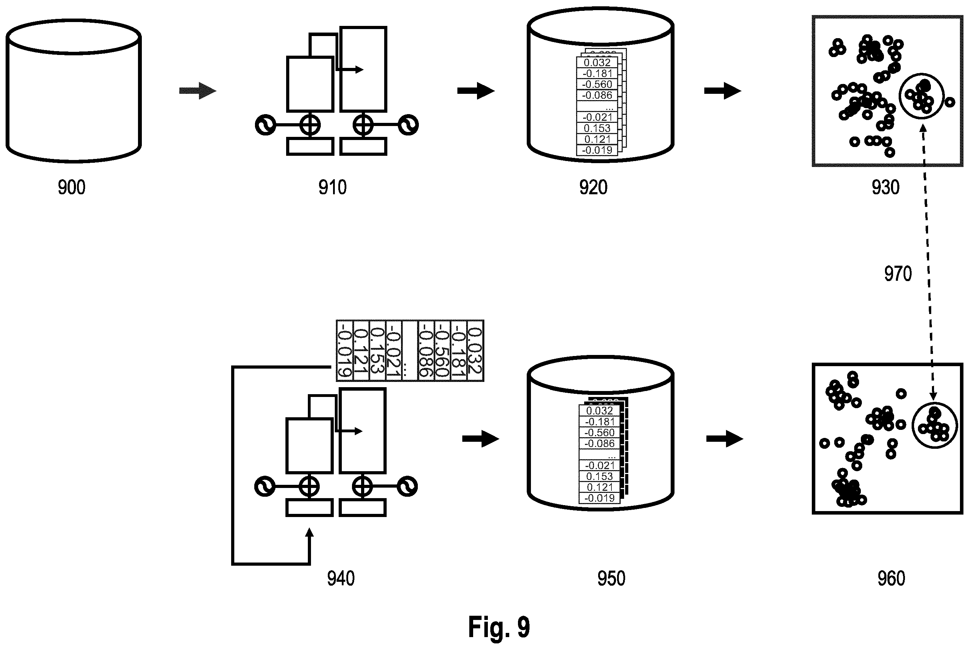

Fig. 5 shows a flow chart of an example of a method for selecting candidate molecules. The method comprises generating 520, using the second machine-learning model, a plurality of embeddings of molecules. The method comprises selecting 530 the plurality of candidate molecules and corresponding embeddings from the plurality of embeddings of molecules according to a selection criterion. This selection process has previously been discussed in connection with Figs. 1a to 2. Additional examples will be given in connection with Figs. 8 and 9. In some examples, the method further comprises training 510 the second machine-learning model using a corpus of tokenized representations of different molecules. In particular, the language model may be trained using a denoising target, and/or the language model may be trained to predict one or more additional tokens given one or more starter tokens. Both the training of the second machine-learning model (as LLM) and the selection of candidate molecules will now be discussed in more detail in connection with Figs. 6 to 9.

For example, the method may be performed by a computer system, e.g., by the system 1010 introduced in connection with Fig. 10 and/or by the computer system 1120 introduced in connection with Fig. 11. For example, the method may be performed by the same computer system, or by a different computer system as the method of Figs. 1a and/or 1b and the method of Fig. 3.

More details and aspects of the method of Fig. 5 are mentioned in connection with the proposed concept or one or more examples described above or below (e.g., Fig. 1a to 4, 6 to 11). The method of Fig. 5 may comprise one or more additional optional features corresponding to one or more aspects of the proposed concept or one or more examples described above or below.

The following description relates to the pre-training of Large Language Models for molecule embeddings. Fig. 6 shows a flow chart of an example of a flow for training a large language model (i.e., the second machine-learning model) on a corpus with sequence data (e.g., sequence data according to nucleotide, peptide or chemical markup language). A large corpus of data 600 comprising nucleotide sequences, protein sequences or chemicals in suitable notation (e.g., SMILE) may be used as a starting point. The items in the corpus may be tokenized 610 as one of individual nucleotides / amino acids as tokens (polynucleotides / polypeptides only), subword tokenization, such as byte-pair encoding or wordpiece tokenization for polypeptides, polynucleotides or low molecular weight chemicals, and chemical residues as tokens (mainly for lower molecular weight chemicals represented as SMILE). Note: For nucleotide sequences (DNA, RNA), it makes the most biological sense to encode trinucleotides (trimers of nucleotides) instead of individual nucleotides as one token as this is the smallest biological unit which can get translated into an amino acid.

A LLM (e.g., the second machine-learning model) 620 is trained on the large corpus as above using a kind of denoising target, such as masked language modeling, see J. Devlin, M.-W. Chang, K. Lee, and K. Toutanova, "BERT: Pre-training of Deep Bidirectional Transformers for Language Understanding,". Alternatively, the pre-training target is to predict the following tokens given a truncated input sequence.

The pre-trained model can then be used to extract features in the form of latent vectors 630 which effectively are semantic (sub-)molecule embeddings. These features can be extracted from the activations computed by the pre-trained model after the last hidden layer of the last transformer block, see A. Vaswani et al., "Attention Is All You Need". For example, the activations may be computed as per-token embeddings. They can be pooled to obtain per (whole) molecule embeddings. This pooling can be global maximum pooling or global average pooling, for example.

More details and aspects of the flow of Fig. 6 are mentioned in connection with the proposed concept or one or more examples described above or below (e.g., Fig. 1a to 5, 7 to 11). The flow of Fig. 6 may comprise one or more additional optional features corresponding to one or more aspects of the proposed concept, or one or more examples described above or below.

The pre-trained model may now be used for sequence generation with a predicted tag embedding (i.e., for generating the plurality of embeddings of molecules). Fig. 7 shows a flow chart of an example of a flow for autoregressive de-novo generation of new tag sequences (i.e., candidate molecules) with selection of suitable candidates in silico (i.e., computationally). In particular, bulk de-novo generation of sequence embeddings may be performed through zero-shot generation.

The (second machine-learning) model pre-trained as outlined above (Fig. 6, 620), which has been trained as a language model, is capable of creating *de novo* sequences 710 in an autoregressive fashion. This means the model gets seeded with one or more tokens and creates the remaining tokens one by one in a loop. Seeding with one token may occur using a specific <start> token. Seeding with multiple tokens may commence with a <start> token and a fixed number of randomly selected tokens (e.g., amino acids, nucleotides). In each iteration of the loop, the input element at time step t may lead to an output *o(t).* Output *o(t)* is then input to the next time step *t+1* and leads to an output *o(t+1).* The loop terminates once an end-of-sequence token was generated, or the maximum length was reached. The generated sequence can be represented as a sequence of token embeddings. From these a "whole protein" embedding can be computed as the arithmetic mean. The protein embedding may be later used to train image models 400, 410 (see Fig. 4) or for comparisons in embedding space 970 (see Fig. 9). The predicted sequence (i.e., the string or token representation of the molecule) may be kept and stored along with the protein embedding.

The "double book-keeping" by using the full architecture may be improved as follows: large language models for text generation usually follow an encoder-decoder paradigm. Transformers fall into this class. Therefore, once trained, the decoder part may (only) be seeded with the entire protein / polynucleotide / chemical compound embedding (which is equivalent to a sentence in natural language). This may be done using the following operations: The pre-trained transformed decoder may be loaded. Then, a sentence embedding vector may be defined or loaded from the de-novo sequences 710 shown in Fig. 7. Then, the decoder state may be initialized with the sentence embedding, and the generated text sequence may be initialized. Subsequently, words may be generated until a stopping criterion is met, by obtaining the next word probabilities from the decoder, sampling the next word from the probability distribution, appending the word to the generated sequence, and updating the state with the newly generated word. After the word generation has terminated, the final generated text sequence may be returned.

This way, a large number of new candidate compounds 710 can be generated, which may then be tested. First, candidates may be selected *in silico* 720 (i.e., computationally) for the desired locus 730 or other desired properties (to select 530 the plurality of candidate molecules and corresponding embeddings from the plurality of embeddings of molecules according to a selection criterion), and their embeddings 740 may be output. Accordingly, the selection criterion may be related to the target property, and in particular to whether the molecule is expected to have a desired quality with respect to the target property. Secondly, the candidates may be tested *in vitro* (experimentally, "in glass", as discussed in connection with Figs. 1a to 2) using chemical synthesis and imaging.

More details and aspects of the flow of Fig. 7 are mentioned in connection with the proposed concept or one or more examples described above or below (e.g., Fig. 1a to 6, 8 to 11). The flow of Fig. 7 may comprise one or more additional optional features corresponding to one or more aspects of the proposed concept, or one or more examples described above or below.

Fig. 8 shows a flow chart of an example of a flow for fine-tuning before autoregressive de novo generation using a classification target or a regression target, to achieve targeted de-Novo generation through fine-tuning. In order to give more control over the desired properties of generated tag candidates, the (second machine-learning) model 620 can be fine-tuned by adding a classification 800 or regression 810 objective. In the case of Fig. 8, the classification or regression objective may be used to select molecules for the plurality of candidate molecules. In case of a specific desired locus, a training set can be created, the training set comprising or consisting of sequences with known locus and the locus, encoded in a suitable fashion (e.g., one-hot). Then a classifier head may be added to the model output. In the simplest case, this can be a logistic regression layer (often referred to as "linear" or "fully connected" in deep learning) with a softmax non-linearity so as to transform the model output into a probability distribution over the classes of interest.

For example, instead of a spatio-temporal distribution D(r), the proposed concept can use cell fate F as the observable to optimize a tag for. For example, one or more additional objectives, in addition to the locus, may be used such as predicted toxicity, solubility in water or hydrophobic media (such as phospholipid bilayers), quantum yield (for fluorophores) or other properties such as binding affinities or biological activity as an inhibitor, activator, allosteric regulator, ligand binding affinity, enzymatic activity. These further objectives can serve as constraints to narrow down the number of predicted target molecules (tags, payloads). Since many molecules can localize to the same locus this additional constraint is beneficial to limit the space of candidate molecules which need to be evaluated.

After fine-tuning the model 800 in supervised fashion with this training set the model can then auto-regressively create de novo sequences as illustrated above in Fig. 7, by creating the candidate molecules (with embeddings) 810 de novo and select 820 some candidate molecules for a desired locus 830 (thus selecting candidate molecules for the plurality of candidate molecules according to a selection criterion, the desired locus). Now the model may specifically generate sequences with the desired locus.

As an alternative to one-hot encoding of loci, sequences with known loci (or other known properties) may be created and passed through the pre-trained model. The output of the last hidden layer is an embedding which captures all properties the model has learned during pre-training, including, e.g., locus. Then the model can be be-tuned using pairs of (sequence; embedding) 810, which biases the model to later generate more selectively new sequences with loci of interest.

De novo-generated molecule embeddings may have the following general properties. As the molecule embeddings predicted by a trained language model are vectors in a semantic molecule space (such as a semantic protein space or nucleotide space or chemical space), the following properties may be expected. For example, spatial proximity in the respective space may encode similarity of molecular properties. Therefore, points in this space corresponding to a particular molecule or tag can be clustered in an unsupervised fashion for visual data inspection. For example, molecule embeddings of tags with similar properties, such as locus, may have a shorter distance than those with dissimilar properties. Hence groups of candidates can be identified using distance metrics, selecting candidate molecules having a low distance to molecules with known desirable properties. Molecular properties may be combined or excluded from one another using simple arithmetic. For example, a molecule can localize to two (or n) loci by adding the vectors of molecule embeddings corresponding to those two (or n) loci. Likewise, different properties may be omitted. If there is a known sequence or molecular residue which is toxic, its respective embedding can be subtracted from an otherwise desirable candidate to suppress this property.

More details and aspects of the flow of Fig. 8 are mentioned in connection with the proposed concept or one or more examples described above or below (e.g., Fig. 1a to 7, 9 to 11). The flow of Fig. 8 may comprise one or more additional optional features corresponding to one or more aspects of the proposed concept, or one or more examples described above or below.

Fig. 9 shows a flow chart of an example of a flow for scoring candidate molecules, such as (predicted) tag candidates, in a latent embedding space. A repository of known tags with known properties 900 may be passed forwardly through the trained large language model (i.e., the second machine-learning model) 910 to compute their embeddings. This new repository of embeddings 920 represents a set of points in the corresponding semantic space 930. A subset of desired properties may be selected, and their center computed, for example using the medoid. In parallel, the large language model (possibly fine-tuned as illustrated above) (autoregressively) may be used to predict 940 *de novo* tag candidate's embeddings 950 (thus generating 520 the plurality of embeddings of molecules) and identify a subset of candidates (candidate molecules) with desired properties 960 (thus selecting 530 the plurality of candidate molecules and corresponding embeddings from the plurality of embeddings of molecules according to a selection criterion) by computing a distance between *de novo* candidates and a cluster of known properties 930, 970. Distance metrics may involve the dot product, cosine similarity, vector norms etc.

A possible prerequisite for the above to work is that the embedding space is continuous (molecules with similar properties correspond to latent vectors in close proximity). Continuity is encouraged by tokenization of the vocabulary using methods such as BPE (Byte-Pair-Encoding) and wordpiece, which are designed that way. It is also encouraged by masked language modeling, which promotes learning context (for classification) by passing outputs through a linear (dense) layer and softmax non-linearity, which treats the prediction as a probability distribution.

One aspect of the proposed concept is a mechanism for predicting not only candidate tags, but also what their distribution in the specimen is going to look like. There is currently mounting evidence that hand-curated categories for, e.g., subcellular localization might be too coarse to fully capture the nuances of a spatio-temporal distribution D(r) of a tag in the specimen [1] (see H. Kobayashi, K. C. Cheveralls, M. D. Leonetti, and L. A. Royer, "Self-Supervised Deep-Learning Encodes High-Resolution Features of Protein Subcellular Localization." bioRxiv, p. 2021.03.29.437595, Mar. 29, 2021. doi: 10.1101/2021.03.29.437595). Using the proposed concept, the expected distribution can be predicted and then tested using microscopy by comparing the predicted to the observed D(r). Thus, there is no explicit need for manually curated labels, even though they may be helpful for visualization and interpretation of the results.

More details and aspects of the flow of Fig. 9 are mentioned in connection with the proposed concept or one or more examples described above or below (e.g., Fig. 1a to 8, 10 to 11). The flow of Fig. 9 may comprise one or more additional optional features corresponding to one or more aspects of the proposed concept, or one or more examples described above or below.

Fig. 10 shows an example of a system that is suitable for performing at least one of the above methods. Fig. 10 shows a schematic diagram of an example of a system 1010, and of an imaging system 1000 comprising a system 1010 and an imaging device 1020 (e.g., a microscope, such as laboratory microscope). The system 1010 comprises one or more processors 1014 and one or more storage devices 1016. Optionally, the system 1010 further comprises one or more interfaces 1012. The one or more processors 1014 are coupled to the one or more storage devices 1016 and to the one or more interfaces 1012. In general, the functionality of the system 1010 may be provided by the one or more processors 1014, in conjunction with the one or more interfaces 1012 (for exchanging data/information with one or more other components of the imaging system and outside the imaging system, such as one or more optical imaging sensors of the imaging device 1020, another system (e.g., a cloud-based system), or a display device of the imaging system), and with the one or more storage devices 1016 (for storing information, such as machine-readable instructions of a computer program being executed by the one or more processors). In general, the functionality of the one or more processors 1014 may be implemented by the one or more processors 1014 executing machine-readable instructions. Accordingly, any feature ascribed to the one or more processors 1014 may be defined by one or more instructions of a plurality of machine-readable instructions. The system 1010 may comprise the machine-readable instructions, e.g., within the one or more storage devices 1016.

Such a system may be used to perform various tasks. For example, the system may be configured to perform the method shown in connection with Figs. 1a and/or 1b. Alternatively, or additionally, the system may be configured to perform the method shown in connection with Fig. 3. Alternatively, or additionally, the system may be configured to perform the method shown in connection with Fig. 5.

In various examples, the system 1010 is used together with the optical imaging device 1020 of the imaging system. In particular, the system 1010 may be co-located with the optical imaging device 1020, which may be a laboratory microscope. Alternatively, the system 1010 may be part of a server (e.g., cloud node), and be coupled to the optical imaging device 1020 via a computer network (e.g., via the internet). In general, the optical imaging device may be configured to generate the set of images being processed. As is evident, the system may be implemented differently, depending on what aspects of the above methods is being performed by the system. For example, the system may be one of a server, a cloud computing node, and a workstation computer.

The one or more interfaces 1012 of the system 1010 may correspond to one or more inputs and/or outputs for receiving and/or transmitting information, which may be in digital (bit) values according to a specified code, within a module, between modules or between modules of different entities. For example, the one or more interfaces 1012 may comprise interface circuitry configured to receive and/or transmit information. The one or more processors 1014 of the system 1010 may be implemented using one or more processing units, one or more processing devices, any means for processing, such as a processor, a computer or a programmable hardware component being operable with accordingly adapted software. In other words, the described function of the one or more processors 1014 may as well be implemented in software, which is then executed on one or more programmable hardware components. Such hardware components may comprise a general-purpose processor, a Digital Signal Processor (DSP), a micro-controller, etc. The one or more storage devices 1016 of the system 1010 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

More details and aspects of the system of Fig. 10 are mentioned in connection with the proposed concept or one or more examples described above or below (e.g., Fig. 1a to 9, 11). The system of Fig. 11 may comprise one or more additional optional features corresponding to one or more aspects of the proposed concept, or one or more examples described above or below.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Embodiments may be based on using a machine-learning model or machine-learning algorithm.

In the above example, the training of the machine-learning model and of the second machine-learning model were explained according to the "supervised learning" training technique.

Apart from supervised or semi-supervised learning, unsupervised learning may be used to train the machine-learning model. In unsupervised learning, (only) input data might be supplied and an unsupervised learning algorithm may be used to find structure in the input data (e.g., by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e., outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g., a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g., be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g., based on the training performed by the machine-learning algorithm). **In** embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g., of the sum of its inputs). The inputs of a node may be used in the function based on a "weight" of the edge or of the node that provides the input. The weight of nodes and/or of edges may be adjusted in the learning process. **In** other words, the training of an artificial neural network may comprise adjusting the weights of the nodes and/or edges of the artificial neural network, i.e., to achieve a desired output for a given input.

Alternatively, the machine-learning model may be a support vector machine, a random forest model or a gradient boosting model. Support vector machines (i.e., support vector networks) are supervised learning models with associated learning algorithms that may be used to analyze data (e.g., in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of Reference Numerals

- 110: Obtaining embeddings of a plurality of candidate molecules
- 120: Determining one or more imaging parameters
- 130: Determining one or more parameters related to sample preparation
- 140: Obtaining one or more images
- 150: Pre-processing the one or more images
- 160: Processing the one or more images or information derived from the one or more images to generate a predicted embeddings
- 170: Comparing the embeddings with the predicted embeddings
- 180: Selecting one or more candidate molecules
- 200: Pre-trained large language model
- 210: Tag candidates
- 215: Semantic embeddings
- 220: Chemical synthesis
- 230: Sample preparation
- 240: Data recordation
- 250: Images
- 260: Trained image recognition model
- 270: Predicted/probable tag embedding
- 280: Comparison
- 290: Fine-tuning
- 310: Obtaining a set of training data
- 312-314: Generating portions of the training data
- 320: Training a machine-learning model
- 400: Training corpus
- 410: Image recognition machine-learning model
- 420: Embedding
- 430: Loss gradient
- 510: Training a second machine-learning model
- 520: Generating a plurality of embeddings of molecules
- 530: Selecting a plurality of candidate molecules
- 600: Large corpus of data
- 610: Tokenization
- 620: Trained Large Language Model
- 630: Latent vector / embedding
- 710: De-novo created sequences
- 720: Selection of candidates
- 730: Desired locus
- 740: Embedding
- 800: Training with classification objective
- 810: Training with regression objective
- 820: Select candidates
- 830: Desired locus
- 900: Tags with known properties
- 910: Large Language Model
- 920: Repository of embeddings
- 930: Set of points in semantic space
- 940: Predict embeddings
- 950: Predicted embeddings
- 960: Subset of candidates with desired properties
- 970: Distance
- 1000: Imaging system
- 1010: System
- 1012: Interface
- 1014: Processor
- 1016: Storage device
- 1020: Optical imaging device / microscope
- 1100: System
- 1110: Microscope
- 1120: Computer system

## Claims

1. A method for processing images of an optical imaging device (240; 520), the method comprising:
obtaining (110; 200) embeddings (215) of a plurality of candidate molecules;
obtaining (140), for each candidate molecule, one or more images (250) of the optical imaging device, the one or more images showing a visual representation of a target property exhibited by the candidate molecule in a biological sample (230);
processing (160; 260), using a machine-learning model, for each candidate molecule, the one or more images and/or information derived from the one or more images to generate a predicted embedding (270) of the candidate molecule, the machine-learning model being trained to output the predicted embedding for an input comprising the one or more images and/or the information derived from the one or more images;
comparing (170; 680) the embeddings (215) of the candidate molecules with the predicted embeddings (270) of the candidate molecules; and
selecting (180) one or more candidate molecules based on the comparison, wherein a candidate molecule is selected if the embedding of the candidate molecule and the predicted embedding of the molecule are sufficiently similar, showing that the candidate molecule behaves as expected with respect to the target property.

2. The method according to claim 1, wherein the target property is one of a spatial distribution, a spatio-temporal distribution, an intensity distribution, and a cell fate.

3. The method according to one of the claims 1 or 2, wherein the candidate molecules are molecules for transporting or sequestering one or more payloads to a target region.

4. The method according to claim 3, wherein the one or more payloads comprise one or more of a fluorophore, a drug for influencing gene expression, a drug for binding as a ligand to a receptor or an enzyme, a drug acting as an allosteric regulator of an enzyme, and a drug competing for a binding site as an antagonist.

5. The method according to one of the claims 1 to 4, wherein the method comprises determining (120) one or more imaging parameters based on the target property of the candidate molecule, and obtaining the one or more images based on the determined one or more imaging parameters,
and/or wherein the method comprises determining (130), for each candidate molecule, one or more parameters related to sample preparation (220) for preparing the sample with the respective candidate molecule and outputting the one or more parameters related to sample preparation.

6. The method according to one of the claims 1 to 5, wherein the machine-learning model is trained to process a set of images showing the biological sample at two or more points in time to output the predicted embedding of the candidate molecule.

7. The method according to one of the claims 1 to 6, wherein the method comprises training (320) the machine-learning model, using supervised learning and using a set of training data, to output the predicted embedding of the candidate molecule based on the one or more images or the information derived from the one or more images.

8. The method according to one of the claims 1 to 7, wherein the method comprises generating (520), using a second machine-learning model, a plurality of embeddings of molecules, and selecting (530) the plurality of candidate molecules and corresponding embeddings from the plurality of embeddings of molecules according to a selection criterion.

9. The method according to claim 8, wherein the method comprises comparing the embeddings of the molecules with one or more embeddings of one or more molecules having a desired quality with respect to the target property, and selecting the plurality of candidate molecules and corresponding embeddings based on the comparison,
or wherein the second machine-learning model has an output indicating a quality of the molecule with respect to the target property, with the selection of the plurality of candidate molecules and corresponding embeddings being based on the output indicating the quality of the molecule with respect to the target property.

10. The method according to one of the claims 8 or 9, wherein the plurality of embeddings are generated autoregressively, by using the second machine-learning model to select, based on a starter token representing a portion of a molecule, one or more additional tokens representing one or more additional portions of the molecule, and generating the respective embeddings by combining the respective starter tokens with the corresponding one or more additional tokens.

11. The method according to one of the claims 8 to 10, wherein the method comprises training (510) the second machine-learning model using the corpus of tokenized representations of different molecules, with the training being performed using the denoising target and/or with the second machine-learning model being trained to predict the one or more additional tokens given the one or more starter tokens.

12. The method according to one of the claims 8 to 11, wherein the second machine-learning model is trained to output an embedding of a molecule based on an input comprising a representation of at least a portion of the molecule.

13. A system (1010) comprising one or more processors (1014) and one or more storage devices (1016), wherein the system is configured to perform the method of one of the claims 1 to 12.

14. Computer program with a program code for performing the method according to one of the claims 1 to 12 when the computer program is run on a processor.

## Patentansprüche

1. Verfahren zur Verarbeitung von Bildern einer optischen Bildgebungsvorrichtung (240; 520), das Verfahren umfassend:
Erhalten (110; 200) von Embeddings (215) einer Vielzahl von Kandidatenmolekülen; Erhalten (140) von einem oder mehreren Bildern (250) der optischen Bildgebungsvorrichtung für jedes Kandidatenmolekül, wobei das eine oder die mehreren Bilder eine visuelle Darstellung einer Zieleigenschaft zeigen, die durch das Kandidatenmolekül in einer biologischen Probe (230) gezeigt wird;
Verarbeiten (160; 260) unter Verwendung eines Maschinenlernmodells für jedes Kandidatenmolekül des einen oder der mehreren Bilder und/oder von Informationen, die von dem einen oder den mehreren Bildern abgeleitet sind, um ein vorhergesagtes Embedding (270) des Kandidatenmoleküls zu erzeugen, wobei das Maschinenlernmodell trainiert ist, das vorhergesagte Embedding für eine Eingabe auszugeben, die das eine oder die mehreren Bilder und/oder die von dem einen oder den mehreren Bildern abgeleiteten Informationen umfasst;
Vergleichen (170; 680) der Embeddings (215) der Kandidatenmoleküle mit den vorhergesagten Embeddings (270) der Kandidatenmoleküle; und
Auswählen (180) von einem oder mehreren Kandidatenmolekülen basierend auf dem Vergleich, wobei ein Kandidatenmolekül ausgewählt wird, wenn das Embedding des Kandidatenmoleküls und das vorhergesagte Embedding des Moleküls hinreichend ähnlich sind, was zeigt, dass sich das Kandidatenmolekül im Hinblick auf die Zieleigenschaft wie erwartet verhält.

2. Verfahren nach Anspruch 1, wobei die Zieleigenschaft eine von einer räumlichen Verteilung, einer räumlich-zeitlichen Verteilung, einer Intensitätsverteilung und einem Zellschicksal ist.

3. Verfahren nach einem der Ansprüche 1 oder 2, wobei die Kandidatenmoleküle Moleküle zum Transportieren oder zum Sequestrieren von einem oder mehreren Nutzlasten zu einer Zielregion sind.

4. Verfahren nach Anspruch 3, wobei der eine oder die mehreren Nutzlasten ein(e) oder mehrere von einem Fluorophor, einem Arzneimittel zur Beeinflussung der Genexpression, einem Arzneimittel zum Binden als Ligand an einen Rezeptor oder ein Enzym, einem Arzneimittel, das als ein allosterischer Regulator eines Enzyms wirkt, und einem Arzneimittel, das als ein Antagonist um eine Bindungsstelle konkurriert, umfassen.

5. Verfahren nach einem der Ansprüche 1 bis 4, wobei das Verfahren das Bestimmen (120) von einem oder mehreren Bildgebungsparametern basierend auf der Zieleigenschaft des Kandidatenmoleküls und das Erhalten des einen oder der mehreren Bilder basierend auf dem einen oder den mehreren bestimmten Bildgebungsparametern umfasst,
und/oder wobei das Verfahren das Bestimmen (130) von einem oder mehreren Parametern, die sich auf die Probenvorbereitung (220) zum Vorbereiten der Probe mit dem jeweiligen Kandidatenmolekül beziehen, für jedes Kandidatenmolekül und das Ausgeben des einen oder der mehreren Parameter, die sich auf die Probenvorbereitung beziehen, umfasst.

6. Verfahren nach einem der Ansprüche 1 bis 5, wobei das Maschinenlernmodell trainiert ist, einen Satz von Bildern zu verarbeiten, die die biologische Probe zu zwei oder mehreren Zeitpunkten zeigen, um das vorhergesagte Embedding des Kandidatenmoleküls auszugeben.

7. Verfahren nach einem der Ansprüche 1 bis 6, wobei das Verfahren das Trainieren (320) des Maschinenlernmodells unter Verwendung von überwachtem Lernen und unter Verwendung eines Satzes von Trainingsdaten umfasst, um das vorhergesagte Embedding des Kandidatenmoleküls basierend auf dem einen oder den mehreren Bildern oder den von dem einen oder den mehreren Bildern abgeleiteten Informationen auszugeben.

8. Verfahren nach einem der Ansprüche 1 bis 7, wobei das Verfahren das Erzeugen (520) einer Vielzahl von Embeddings von Molekülen unter Verwendung eines zweiten Maschinenlernmodells und das Auswählen (530) der Vielzahl von Kandidatenmolekülen und entsprechender Embeddings aus der Vielzahl von Embeddings von Molekülen nach einem Auswahlkriterium umfasst.

9. Verfahren nach Anspruch 8, wobei das Verfahren das Vergleichen der Embeddings der Moleküle mit einem oder mehreren Embeddings von einem oder mehreren Molekülen mit einer gewünschten Qualität im Hinblick auf die Zieleigenschaft und das Auswählen der Vielzahl von Kandidatenmolekülen und entsprechender Embeddings basierend auf dem Vergleich umfasst, oder wobei das zweite Maschinenlernmodell eine Ausgabe hat, die eine Qualität des Moleküls im Hinblick auf die Zieleigenschaft angibt, wobei das Auswählen der Vielzahl von Kandidatenmolekülen und entsprechender Embeddings auf der Ausgabe basiert, die die Qualität des Moleküls im Hinblick auf die Zieleigenschaft angibt.

10. Verfahren nach einem der Ansprüche 8 oder 9, wobei die Vielzahl von Embeddings autoregressiv erzeugt wird, indem das zweite Maschinenlernmodell verwendet wird, um basierend auf einem Start-Token, das einen Abschnitt eines Moleküls repräsentiert, ein oder mehrere zusätzliche Token auszuwählen, die einen oder mehrere zusätzliche Abschnitte des Moleküls repräsentieren, und die jeweiligen Embeddings durch Kombinieren der jeweiligen Start-Token mit dem entsprechenden einen oder den entsprechenden mehreren zusätzlichen Token erzeugt werden.

11. Verfahren nach einem der Ansprüche 8 bis 10, wobei das Verfahren das Trainieren (510) des zweiten Maschinenlernmodells unter Verwendung des Korpus tokenisierter Repräsentationen verschiedener Moleküle umfasst, wobei das Training unter Verwendung des Entrauschungsziels durchgeführt wird und/oder wobei das zweite Maschinenlernmodell trainiert ist, das eine oder die mehreren zusätzlichen Token bei Vorgabe des einen oder der mehreren Start-Token vorherzusagen.

12. Verfahren nach einem der Ansprüche 8 bis 11, wobei das zweite Maschinenlernmodell trainiert ist, ein Embedding eines Moleküls basierend auf einer Eingabe auszugeben, die eine Repräsentation von zumindest einem Abschnitt des Moleküls umfasst.

13. System (1010), umfassend einen oder mehrere Prozessoren (1014) und eine oder mehrere Speichervorrichtungen (1016), wobei das System konfiguriert ist, das Verfahren nach einem der Ansprüche 1 bis 12 durchzuführen.

14. Computerprogramm mit einem Programmcode zur Durchführung des Verfahrens nach einem der Ansprüche 1 bis 12, wenn das Computerprogramm auf einem Prozessor ausgeführt wird.

## Revendications

1. Procédé pour traiter des images d'un dispositif d'imagerie optique (240 ; 520), le procédé comprenant les étapes consistant à :
obtenir (110 ; 200) des représentations intégrées (215) d'une pluralité de molécules candidates ;
obtenir (140), pour chaque molécule candidate, une ou plusieurs images (250) du dispositif d'imagerie optique, la ou les images montrant une représentation visuelle d'une propriété cible présentée par la molécule candidate dans un échantillon biologique (230) ;
traiter (160 ; 260), en utilisant un modèle d'apprentissage automatique, pour chaque molécule candidate, la ou les images et/ou des informations dérivées de la ou des images pour générer une représentation intégrée prédite (270) de la molécule candidate, le modèle d'apprentissage automatique étant entraîné pour délivrer en sortie la représentation intégrée prédite pour une entrée comprenant la ou les images et/ou les informations dérivées de la ou des images ;
comparer (170 ; 680) les représentations intégrées (215) des molécules candidates avec les représentations intégrées prédites (270) des molécules candidates ; et
sélectionner (180) une ou plusieurs molécules candidates sur la base de la comparaison, dans lequel une molécule candidate est sélectionnée si la représentation intégrée de la molécule candidate et la représentation intégrée prédite de la molécule sont suffisamment similaires, ce qui montre que la molécule candidate se comporte comme prévu vis-à-vis de la propriété cible.

2. Procédé selon la revendication 1, dans lequel la propriété cible est l'une des suivantes : une distribution spatiale, une distribution spatio-temporelle, une distribution d'intensité et un destin cellulaire.

3. Procédé selon l'une des revendications 1 ou la revendication 2, dans lequel les molécules candidates sont des molécules pour transporter ou séquestrer une ou plusieurs charges utiles vers une région cible.

4. Procédé selon la revendication 3, dans lequel la ou les charges utiles comprennent un ou plusieurs des éléments suivants : un fluorophore, un médicament pour influencer l'expression génique, un médicament pour se lier en tant que ligand à un récepteur ou à une enzyme, un médicament agissant comme régulateur allostérique d'une enzyme et un médicament entrant en compétition pour un site de liaison en tant qu'antagoniste.

5. Procédé selon l'une des revendications 1 à 4, dans lequel le procédé comprend la détermination (120) d'un ou plusieurs paramètres d'imagerie sur la base de la propriété cible de la molécule candidate, et obtenir la ou les images sur la base du ou des paramètres d'imagerie déterminés,
et/ou dans lequel le procédé comprend la détermination (130), pour chaque molécule candidate, d'un ou plusieurs paramètres liés à la préparation de l'échantillon (220) pour préparer l'échantillon avec la molécule candidate respective et la délivrance en sortie du ou des paramètres liés à la préparation de l'échantillon.

6. Procédé selon l'une des revendications 1 à 5, dans lequel le modèle d'apprentissage automatique est entraîné pour traiter un ensemble d'images montrant l'échantillon biologique à deux ou plusieurs points dans le temps pour délivrer en sortie la représentation intégrée prédite de la molécule candidate.

7. Procédé selon l'une des revendications 1 à 6, dans lequel le procédé comprend l'entraînement (320) du modèle d'apprentissage automatique, en utilisant un apprentissage supervisé et en utilisant un ensemble de données d'apprentissage, pour délivrer en sortie la représentation intégrée prédite de la molécule candidate sur la base de la ou des images ou des informations dérivées de la ou des images.

8. Procédé selon l'une des revendications 1 à 7, dans lequel le procédé comprend la génération (520), en utilisant un second modèle d'apprentissage automatique, d'une pluralité de représentations intégrées de molécules, et la sélection (530) de la pluralité de molécules candidates et des représentations intégrées correspondantes à partir de la pluralité de représentations intégrées de molécules selon un critère de sélection.

9. Procédé selon la revendication 8, dans lequel le procédé comprend la comparaison des représentations intégrées des molécules avec une ou plusieurs représentations intégrées d'une ou plusieurs molécules présentant une qualité souhaitée par rapport à la propriété cible, et la sélection de la pluralité de molécules candidates et des représentations intégrées correspondantes sur la base de la comparaison, ou dans lequel le second modèle d'apprentissage automatique présente une sortie indiquant une qualité de la molécule par rapport à la propriété cible, la sélection de la pluralité de molécules candidates et des représentations intégrées correspondantes étant effectuée sur la base de la sortie indiquant la qualité de la molécule par rapport à la propriété cible.

10. Procédé selon l'une des revendications 8 ou la revendication 9, dans lequel la pluralité de représentations intégrées est générée de manière autorégressive, en utilisant le second modèle d'apprentissage automatique pour sélectionner, sur la base d'un jeton de départ représentant une portion d'une molécule, un ou plusieurs jetons supplémentaires représentant une ou plusieurs portions supplémentaires de la molécule, et en combinant les jetons de départ respectifs avec le ou les jetons supplémentaires correspondants pour générer les représentations intégrées respectives.

11. Procédé selon l'une des revendications 8 à 10, dans lequel le procédé comprend l'entraînement (510) du second modèle d'apprentissage automatique en utilisant le corpus de représentations sous forme de jetons de différentes molécules, l'entraînement étant effectué en utilisant la cible de débruitage et/ou en entraînant le second modèle d'apprentissage automatique à prédire le ou les jetons supplémentaires étant donné le ou les jetons de départ.

12. Procédé selon l'une des revendications 8 à 11, dans lequel le second modèle d'apprentissage automatique est entraîné pour délivrer en sortie une représentation intégrée d'une molécule sur la base d'une entrée comprenant une représentation d'au moins une portion de la molécule.

13. Système (1010) comprenant un ou plusieurs processeurs (1014) et un ou plusieurs dispositifs de stockage (1016), dans lequel le système est configuré pour exécuter le procédé de l'une des revendications 1 à 12.

14. Programme informatique avec un code de programme pour exécuter le procédé selon l'une des revendications 1 à 12 lorsque le programme informatique est exécuté sur un processeur.
